# EUROPEAN PATENT SPECIFICATION

(11) **EP 3 138 924 B1**
(45) Date of publication and mention of the grant of the patent: **23.10.2019**
(21) Application number: 16158249.9
(22) Date of filing: 02.03.2016
(51) Int. Cl.: C12Q 1/6806, C09B 57/10, C07F 15/00, C09B 69/00, C12N 15/87

(54) **APPLICATION OF RUTHENIUM COMPLEXES AS NUCLEIC ACID VECTORS OF TARGET CELL NUCLEUSES**
VERWENDUNG VON RUTHENIUM-KOMPLEXEN ALS NUKLEINSÄUREVEKTOREN FÜR ZIELZELLKERNE
APPLICATION DE COMPLEXES DE RUTHÉNIUM EN TANT QUE VECTEURS D'ACIDE NUCLÉIQUE DE NOYAUX DE CELLULE CIBLE

(30) Priority: 02.09.2015 CN 201510559959
(43) Date of publication of application: 08.03.2017
(73) Proprietor: Guangdong Pharmaceutical University, Guangdong 510006 (CN)
(72) Inventor: MEI, Wenjie, Guangzhou, Guangdong 510006 (CN)
(74) Representative: Gassner, Birgitta

(56) References cited:
- CN-A- 103 788 134
- CN-A- 105 238 814
- US-A- 5 157 032
- SHI S ET AL: "Synthesis, characterization and DNA-binding of novel chiral complexes DELTA- and LAMBDA-[Ru(bpy)2L]<2+> (L=o-mopip and p-mopip)", JOURNAL OF INORGANIC BIOCHEMISTRY, ELSEVIER INC, US, vol. 100, no. 3, 1 March 2006 (2006-03-01) , pages 385-395, XP025038400, ISSN: 0162-0134, DOI: 10.1016/J.JINORGBIO.2005.12.005 [retrieved on 2006-03-01]
- BIN SUN ET AL: "DNA Condensation Induced by Ruthenium(II) Polypyridyl Complexes [Ru(bpy)2(PIPSH)]2+ and [Ru(bpy)2(PIPNH)]2+", INORGANIC CHEMISTRY, vol. 48, no. 11, 10 April 2009 (2009-04-10), pages 4637-4639, XP055330745, EASTON, US ISSN: 0020-1669, DOI: 10.1021/ic900102r
- SATISH S. BHAT ET AL: "Efficient DNA Condensation Induced by Ruthenium(II) Complexes of a Bipyridine-Functionalized Molecular Clip Ligand", CHEMISTRY - A EUROPEAN JOURNAL., vol. 18, no. 51, 14 December 2012 (2012-12-14), pages 16383-16392, XP055330761, WEINHEIM, DE ISSN: 0947-6539, DOI: 10.1002/chem.201200407
- Hao Yin ET AL: "Non-viral vectors for gene-based therapy", NATURE REVIEWS GENETICS, vol. 15, no. 8, 15 July 2014 (2014-07-15), pages 541-555, XP055240438, GB ISSN: 1471-0056, DOI: 10.1038/nrg3763

### SEQUENCE LISTING

<110> Guangdong Pharmaceutical University
<120> Application of ruthenium complexes as nucleic acid vectors of target cell nucleuses
<130> EP2051
<150> CN201510559959.3
   <151> 2015-09-02
<160> 7
<170> BiSSAP 1.3
<210> 1
   <211> 22
   <212> DNA
   <213> Artificial Sequence
<220>
   <223> c-myc Pu22
<400> 1
   tgagggtggg gagggtgggg aa 22
<210> 2
   <211> 27
   <212> DNA
   <213> Artificial Sequence
<220>
   <223> Bcl-2 Pu27
<400> 2
   cgggcgcggg aggaaggggg cgggagc 27
<210> 3
   <211> 21
   <212> DNA
   <213> Artificial Sequence
<220>
   <223> c-Kit 1
<400> 3
   gggagggcgc tgggaggagg g 21
<210> 4
   <211> 32
   <212> DNA
   <213> Artificial Sequence
<220>
   <223> K-ras
<400> 4
   gcggtgtggg aagagggaag agggggaggc ag 32
<210> 5
   <211> 26
   <212> DNA
   <213> Artificial Sequence
<220>
   <223> AS1411 sequence
<400> 5
   ggtggtggtg gttgtggtgg tggtgg 26
<210> 6
   <211> 22
   <212> RNA
   <213> Artificial Sequence
<220>
   <223> miR-21RNA sequence
<400> 6
   uagcuuauca gacugauguu ga 22
<210> 7
   <211> 18
   <212> RNA
   <213> Artificial Sequence
<220>
   <223> PAR-1 siRNA
<400> 7
   agauuagucu ccaucaua 18

## Description

### Technical Field of the Invention

The invention relates to a novel application of ruthenium complexes, especially to a novel use of ruthenium complexes as nucleic acid vectors.

### Background of the Invention

Gene therapy techniques used in treating diseases, such as tumors, have increasingly drawn attentions and concerns of a large number of researchers. The so-called gene therapy means that transfecting some functional genetic materials, including siRNA, mRNA, miRNA, DNA, nucleic acid aptamers and promoter region sequences of cancer genes, into cells and making them express in the cells to achieve objectives of treating diseases finally. A key factor of gene therapy is to transfect therapeutic genes into cells by using safe and efficient vectors. Generally, viral vectors such as RNA viruses or DNA viruses, or non-viral vectors including calcium phosphate precipitation, lipofection or microinjection and the like, to transfect therapeutic genes (H. Yin, R.L. Kanasty, A.A. Eltoukhy, A.J. Vegas, J.R. Dorkin, D.G. Anderson, Non-viral vectors for gene-based therapy, Nature Rev., 15, 541-555, 2014; Viktoriya Sokolova and Matthias Epple, Inorganic Nanoparticles as Carriers of Nucleic Acids into Cells, Angew. Chem. Int. Ed. 2008, 47, 1382 - 1395; S. Huo, S. Jin, X. Ma, X. Xue, K. Yang, A. Kumar, P.C. Wang, J. Zhang, Z. Hu, X.-J. Liang, Ultrasmall gold nano particles as carriers for nucleus-based gene therapy due to size-dependent nuclear entry, ACS NANO, 8(6), 5852-5862, 2014). However, due to their defects and deficiencies, clinical uses of these methods are still greatly limited.

In recent years, the study of using ruthenium (II) complexes as gene vectors has aroused widespread interests of many researchers. Kumbhar et al reported an application of ruthenium polypyridine complexes as gene vectors (S.S. Bhat, A.S. Kumbhar, A.K. Kumbhar, A. Khan, P. Lonnecke, Ruthenium(II) polypyridyl complexes as carriers for DNA delivery, Chem. Comm., 2011, 47, 11068-1070); Chao Hui et al reported a method of using ruthenium (II) polypyridyl complexes as gene vectors (B. Yu, Y. Chen, C. Ouyang, H. Huang, L. Ji and H. Chao; A luminescent tetranuclear ruthenium(II) complex as a tracking non-viral gene vector, Chem. Commun., 2013, 49, 810-812). However, the ruthenium complexes used in these reports have relatively large molecular weight with relatively complicated synthesis processes, and have limited effects when used as gene vectors. In addition, all above reports on ruthenium complexes and their optical isomers didn't relate to their uses as gene vectors of target cell nucleuses. Since replication and transcription of genes mainly takes place in cell nucleuses, therefore efficiency of self-replication and transcription of genes can be improved by nucleic acid-targeted delivering to cell nucleuses, thereby better therapeutic effects can be obtained.

S. S. Bhat, A. S. Kumbhar, A. A. Kumbhar and A. Khan, Efficient DNA Condensation Induced by Ruthenium(II) Complexes of a Bipyridine-Functionalized Molecular Clip Ligand, Chem. Eur. J. 2012, 18, 16383-16392, deals with the cellular uptake of ruthenium complexes with GFP(Green Fluorescent Protein)-labeled DNA; in particular, nuclear localization is reported.

### Summary of the Invention and the further Disclosure

The present invention relates to a non-therapeutic application of ruthenium-nucleic acid complexes as nucleic acid vectors as defined in claim 1 and a method of preparing a ruthenium coordination complex-nucleic acid complex as defined in claim 5.

Of the general disclosure which is presented in the following, protection is sought only for those parts which fall within the scope of the claims.

One object of the present disclosure is to provide an application of ruthenium complexes as nucleic acid vectors, specifically ruthenium-DNA complexes for use as nucleic acid vectors.

Experimental data shows that ruthenium (II) complexes having the following general formula may be effectively combined with nucleic acid sequence and may effectively change morphologies of long sequence (e.g., more than 50 bp) nucleic acids to make the nucleic acids be effectively delivered into viable cells via transmembrane transport and be well located within cell nucleuses, thus greatly improving transport efficiency of the nucleic acids. Based on this property, nucleic acid sequences can be conveniently transported into cells for gene therapy or fluorescent tracking, etc.

The ruthenium (II) complex has the following general formula: 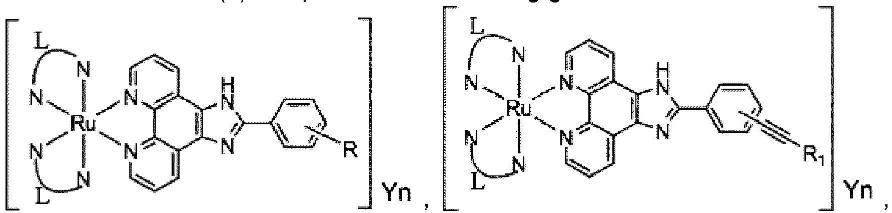 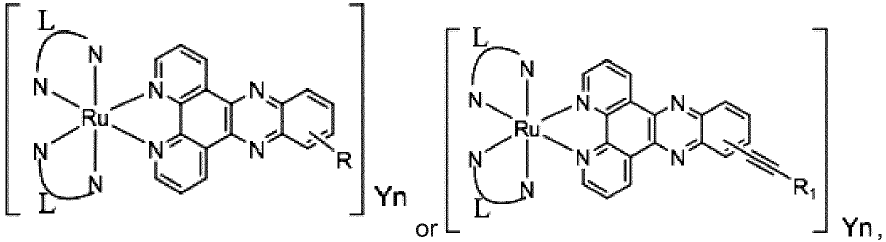 wherein:
**L** is an auxiliary ligand having a structural formula of: 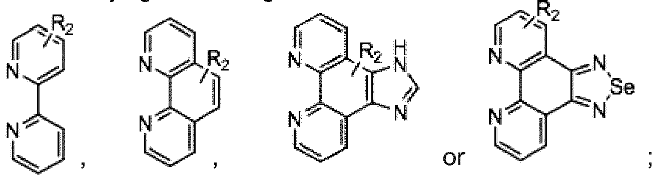
**R** is independently selected from substituted alkyl, substituted phenyl, substituted pyridyl, substituted furyl, substituted thiazole and substituted pyrrole, wherein substituted group is optionally selected from hydroxyl, nitro, halogen, amino, carboxyl, cyano, mercapto, C₃-C₈ cycloalkyl, SO₃H, C₁-C₆ alkyl, C₂-C₆ alkenyl, C₂-C₆ alkynyl, hydroxyl(C₁-C₆)alkyl, amino(C₁-C₆)alkyl, CO₂R', CONR'R', COR', SO₂R'R', (C₁-C₆)alkoxyl, (C₁-C₆)alkylsulphide, -N=NR', NR'R' or trifluoro(C₁-C₆)alkyl; wherein **R'** is selected from H, C₁-C₆ alkyl or phenyl;
**R₁** is independently selected from H, hydroxyl, trimethylsilyl, C₁-C₆ alkyl or substituted C₁-C₆ alkyl, phenyl or substituted phenyl, pyridyl or substituted pyridyl, furyl or substituted furyl, pyrryl or substituted pyrryl, thiazolyl or substituted thiazolyl; wherein the position of R₁ substituted ethynyl may be ortho-position, meta-position or para-position on the benzene ring; the number of substituted ethynyl is 1, 2 or more;
**R₂** is independently selected from H, methyl, methoxyl, nitro-group and halogen; **Y** is an ion or acidic radical ion which makes the whole ruthenium(II) complex be electrically neutral, n is the number of ions or acidic radical ions which makes the whole ruthenium(II) complex be electrically neutral.

The length of the nucleic acid sequence is at least 4 bp. In particular, the length of the nucleic acid sequence is not longer than 3,000 bp.

As a further improvement of above application, the ruthenium complex is a single chiral isomer thereof.

As a further improvement of above application, the length of the nucleic acid sequence is not longer than 3,000 bp. The nucleic acid may be selected from c-myc promoter region DNA, C-kit promoter region DNA, bcl-2 promoter region DNA, miR-21, DNA of AS1411, SiRNA, microRNA, aptamer and mRNA.

As a further improvement of above application, the nucleic acid is a fluorophore-labeled nucleic acid.

The present disclosure also provides an application of a ruthenium coordination complex-nucleic acid complex used as a fluorescent probe, wherein the ruthenium complex is as previously described.

The present disclosure also provides a method of preparing ruthenium coordination complex-nucleic acid complex, comprising the following steps:
the ruthenium complex is uniformly mixed with the nucleic acid solution, then the mixture obtained is heated to a temperature ranged from 70 to 100°C and maintained at the temperature for 30 s to 30 min;
free ruthenium complexes are removed after cooling to form a ruthenium coordination complex-nucleic acid complex;
wherein the ruthenium complex has a structural formula according to any of claims 1-3 and the length of the nucleic acid is not shorter than 4 bp.

As a further improvement of above method, the heating is performed by a microwave.

As a further improvement of above method, the nucleic acid is a fluorophore-labeled nucleic acid.

The present disclosure has the following advantageous effects:
The method for preparing a ruthenium coordination complex-nucleic acid complex in accordance with the present disclosure may provide a stable ruthenium coordination complex-nucleic acid complex in a more efficient manner and may provide many better effects.

### Brief Description of the Drawings

Figs.1-41 are structural formulas of the ruthenium coordination complexes used in examples 1-41 (if any) and TEMs of the complexes obtained by combining these coordination complexes with DNA respectively.
Fig. 42 shows distribution and location of the ruthenium coordination complex - nucleic acid (FAM fluorescence-labeled) complex [Ru(bpy)₂pBEPIP)(ClO₄)₂: FAM-c-myc pu22 DNA=1:1) in hepatic carcinoma HepG2 cells observed under a fluorescence microscope.
Fig. 43 shows distribution and location of the ruthenium coordination complex - nucleic acid (FAM fluorescence-labeled) complex [Ru(bpy)₂pBEPIP)(ClO₄)₂: FAM-c-myc pu22 DNA=1:1) in cervical carcinoma Hela cells observed under a fluorescence microscope.
Fig. 44 shows distribution and location of the ruthenium coordination complex - nucleic acid (FAM fluorescence-labeled) complex [Ru(bpy)₂pBEPIP)(ClO₄)₂:FAM-c-myc pu22 DNA=1:1) in breast cancer MCF-7 cells observed under a fluorescence microscope.
Fig. 45 shows distribution and location of the ruthenium coordination complex - nucleic acid (FAM fluorescence-labeled) complex [*Λ*-Ru(bpy)₂pBEPIP](ClO₄)₂: FAM-c-myc pu22 DNA=1:1) in hepatic carcinoma HepG2 cells observed under a confocal laser scanning microscope.
Fig. 46 shows distribution and location of the ruthenium coordination complex - nucleic acid (FAM fluorescence-labeled) complex [*Λ*-Ru(bpy)₂pBEPIP](ClO₄)₂:FAM-c-myc pu22 DNA=1:1) in hepatic carcinoma MCF-7 cells observed under a confocal laser scanning microscope.
Fig. 47 shows distribution and location of the ruthenium coordination complex - nucleic acid (FAM fluorescence-labeled) complex [*Δ*-Ru(bpy)₂pBEPIP)(ClO₄)₂: FAM-c-myc pu22 DNA=1:1) in hepatic carcinoma HepG2 cells observed under a fluorescence microscope.
Fig. 48 shows distribution and location of the ruthenium coordination complex - nucleic acid (FAM fluorescence-labeled) complex [*Λ*-Ru(bpy)₂pEPIP](ClO₄)₂: FAM-AS1411 DNA=1:1) in hepatic carcinoma HepG2 cells observed under a fluorescence microscope.

### Detailed Description of the Invention and the further Disclosure

The ruthenium complexes used in the present disclosure may be synthetized by methods of the applications (CN103709202A and CN103788134A) previously filed by the inventor or other known methods.

The technical schemes of the present disclosure will be further described hereafter in accompany with detailed examples.

The nucleic acid sequences used in the following examples are as follows:
c-myc Pu22: 5'- TGAGGGTGGGGAGGGTGGGGAA-3' (SEQ ID NO : 1)
Bcl-2 Pu27 : 5'- CGGGCGCGGGAGGAAGGGGGCGGGAGC-3' (SEQ ID NO : 2)
c-Kit 1 : 5'-GGGAGGGCGCTGGGAGGAGGG-3' (SEQ ID NO : 3)
K-ras : 5'-GCGGTGTGGGAAGAGGGAAGAGGGGGAGGCAG-3' (SEQ ID NO : 4)
Telomere DNA sequence: 5'- TTAGGG-3'
AS1411 sequence: 5'-GGTGGTGGTGGTTGTGGTGGTGGTGG-3' (SEQ ID NO : 5)
miR-21RNA sequence: 5'-UAGCUUAUCAGACUGAUGUUGA-3' (SEQ ID NO : 6)
PAR-1 siRNA: 5'-AGAUUAGUCUCCAUCAUA-3' (SEQ ID NO : 7) .

### Example 1: Preparation of [Ru(bpy)₂pBEPIP](ClO₄)₂-c-myc Promoter Region DNA Complex

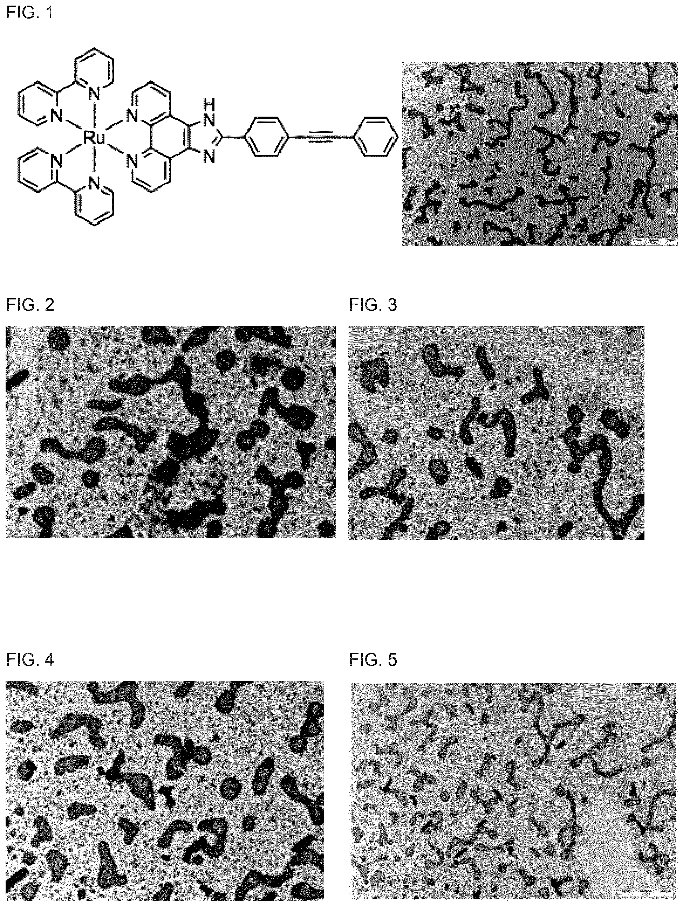

1 mM [Ru(bpy)2pBEPIP](ClO₄)₂ (Fig.1A) is uniformly mixed with 1 mM c-myc Pu22 solution in a ratio of 1:1, then the mixture obtained is heated to 90°C and maintained at the temperature for 0.5 to 10 min, then the mixture is naturally cooled down to room temperature and left to stand for 24 h at 4°C. The reaction mixture is dialyzed against distilled water to remove the polypyridyl ruthenium (II) complexes unloaded onto the nucleic acid, thereby to form a polypyridyl ruthenium (II) coordination complex - nucleic acid complex. It has been found by a TEM detection that the ruthenium complex facilitates self-assembly of the c-myc Pu22 sequence to form a nanotube structure (Fig.1B).

### Example 2: Preparation of [Ru(bpy)₂pBEPIP](ClO₄)₂-Telomere DNA Complex

1 mM [Ru(bpy)2pBEPIP](ClO₄)₂ is uniformly mixed with 1 mM DNA (5'-TTAGGG-3') solution in a ratio of 1:1, then the mixture obtained is heated to 90°C and maintained at the temperature for 0.5 to 10 min, then the mixture is naturally cooled down to room temperature and left to stand for 24 h at 4°C. The reaction mixture is dialyzed against distilled water to remove the polypyridyl ruthenium (II) complexes unloaded onto the nucleic acid, thereby to form a polypyridyl ruthenium (II) coordination complex - nucleic acid complex. It has been found by a TEM detection that the ruthenium complex facilitates self-assembly of the telomere DNA sequence to form an analogous nanotube structure (Fig.2).

### Example 3: Preparation of [Ru(bpy)₂pBEPIP](ClO₄)₂-bcl-2 Promoter Region DNA Complex

1 mM [Ru(bpy)₂pBEPIP](ClO₄)₂ is uniformly mixed with 1 mM Bcl-2 pu27 solution in a ratio of 1:1, then the mixture obtained is heated to 90°C and maintained at the temperature for 0.5 to 10 min, then the mixture is naturally cooled down to room temperature and left to stand for 24 h at 4°C. The reaction mixture is dialyzed against distilled water to remove the polypyridyl ruthenium (II) complexes unloaded onto the nucleic acid, thereby to form a polypyridyl ruthenium (II) coordination complex - nucleic acid complex. It has been found by a TEM detection that the ruthenium complex facilitates self-assembly of the telomere DNA sequence to form an analogous nanotube structure (Fig.3).

### Example 4: Preparation of [Ru(bpy)₂pBEPIP](ClO₄)₂-c-kit Promoter Region DNA Nano-complex

1 mM [Ru(bpy)₂pBEPIP](ClO₄)₂ is uniformly mixed with 1 mM c-kit pu27 solution in a ratio of 1:1, then the mixture obtained is heated to 90°C under microwave radiation and maintained at the temperature for 0.5 to 10 min, then the mixture is naturally cooled down to room temperature. The reaction mixture is dialyzed against distilled water to remove the polypyridyl ruthenium (II) complexes unloaded onto the nucleic acid, thereby to form a polypyridyl ruthenium (II) coordination complex - nucleic acid complex. It has been found by a TEM detection that the ruthenium complex facilitates self-assembly of the telomere DNA sequence to form an analogous nanotube structure (Fig.4).

### Example 5: Preparation of [Ru(bpy)₂pBEPIP](ClO₄)₂-K-Ras Promoter Region DNA Nano-complex

1 mM [Ru(bpy)₂pBEPlP](ClO₄)₂ is uniformly mixed with 1 mM K-Ras pu32 solution in a ratio of 1:1, then the mixture obtained is heated to 90°C under microwave radiation and maintained at the temperature for 0.5 to 10 min, then the mixture is naturally cooled down to room temperature. The reaction mixture is dialyzed against distilled water to remove the polypyridyl ruthenium (II) complexes unloaded onto the nucleic acid, thereby to form a polypyridyl ruthenium (II) coordination complex - nucleic acid complex. It has been found by a TEM detection that the ruthenium complex facilitates self-assembly of the telomere DNA sequence to form an analogous nanotube structure (Fig.5).

### Example 6: Preparation of [Ru(bpy)₂pBEPIP]Cl₂-Aptamer AS1411 DNA Nano-complex

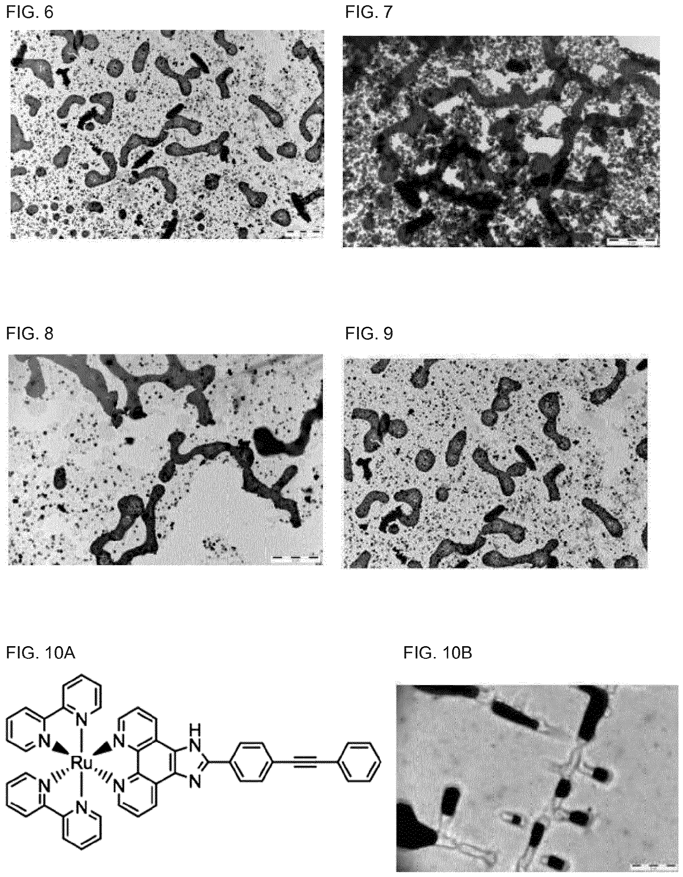

1 mM [Ru(bpy)₂pBEPIP]Cl₂ is uniformly mixed with 1 mM AS1411 DNA solution in a ratio of 1:1, then the mixture obtained is heated to 90°C under microwave radiation and maintained at the temperature for 0.5 to 10 min, then the mixture is naturally cooled down to room temperature. The reaction mixture is dialyzed against distilled water to remove the polypyridyl ruthenium (II) complexes unloaded onto the nucleic acid, thereby to form a polypyridyl ruthenium (II) coordination complex - nucleic acid complex. It has been found by a TEM detection that the ruthenium complex facilitates self-assembly of the telomere DNA sequence to form an analogous nanotube structure (Fig.6).

### Example 7: Preparation of [Ru(bpy)₂pBEPIP]Cl₂-CT-DNA Nano-complex

1 mM [Ru(bpy)₂pBEPIP]Cl₂ is uniformly mixed with 1 mM CT-DNA solution in a ratio of 1:1, then the mixture obtained is heated to 90°C under microwave radiation and maintained at the temperature for 0.5 to 10 min, then the mixture is naturally cooled down to room temperature and left to stand for 24 h at 4°C. The reaction mixture is dialyzed against distilled water to remove the polypyridyl ruthenium (II) complexes unloaded onto the nucleic acid, thereby to form a polypyridyl ruthenium (II) coordination complex - nucleic acid complex. It has been found by a TEM detection that the ruthenium complex facilitates self-assembly of the telomere DNA sequence to form an analogous nanotube structure (Fig.7).

### Example 8: Preparation of [Ru(bpy)₂pBEPIP](ClO₄)₂-miR-21 Nano-complex

1 mM [Ru(bpy)₂pBEPIP](ClO₄)₂ is uniformly mixed with 10 mM miR-21 solution in a ratio of 1:1, then the mixture obtained is heated to 90°C under microwave radiation and maintained at the temperature for 0.5 to 10 min, then the mixture is naturally cooled down to room temperature and left to stand for 24 h at 4°C. The reaction mixture is dialyzed against distilled water to remove the polypyridyl ruthenium (II) complexes unloaded onto the nucleic acid, thereby to form a polypyridyl ruthenium (II) coordination complex - nucleic acid complex. It has been found by a TEM detection that the ruthenium complex facilitates self-assembly of the telomere DNA sequence to form an analogous nanotube structure (Fig.8).

### Example 9: Preparation of [Ru(bpy)₂pBEPIP](ClO₄)₂-PAR-1 SiRNA Nano-complex

10 mM [Ru(bpy)₂pBEPIP](ClO₄)₂ is uniformly mixed with 1 mM SiRNA solution in a ratio of 10:1, then the mixture obtained is heated to 90°C under microwave radiation and maintained at the temperature for 0.5 to 10 min, then the mixture is naturally cooled down to room temperature and left to stand for 24 h at 4°C. The reaction mixture is dialyzed against distilled water to remove the polypyridyl ruthenium (II) complexes unloaded onto the nucleic acid, thereby to form a polypyridyl ruthenium (II) coordination complex - nucleic acid complex. It has been found by a TEM detection that the ruthenium complex facilitates self-assembly of the telomere DNA sequence to form an analogous nanotube structure (Fig.9).

### Example 10: Preparation of Λ-[Ru(bpy)₂pBEPIP](ClO₄)₂-c-myc pu22 DNA Nano-complex

1 mM Λ- [Ru(bpy)₂pBEPIP](ClO₄)₂ (Fig.10A) is uniformly mixed with 1 mM c-myc Pu22 DNA solution in a ratio of 1:1, then the mixture obtained is heated to 90°C and maintained at the temperature for 0.5 to 10 min, then the mixture is naturally cooled down to room temperature and left to stand for 24 h at 4°C. The reaction mixture is dialyzed against distilled water to remove the polypyridyl ruthenium (II) complexes unloaded onto the nucleic acid, thereby to form a polypyridyl ruthenium (II) coordination complex - nucleic acid complex. It has been found by a TEM detection that the ruthenium complex facilitates self-assembly of the telomere DNA sequence to form an analogous nanotube structure (Fig.10B).

### Example 11: Preparation of Δ-[Ru(bpy)₂pBEPIP](ClO₄)₂-c-myc Promoter Region DNA Nano-complex

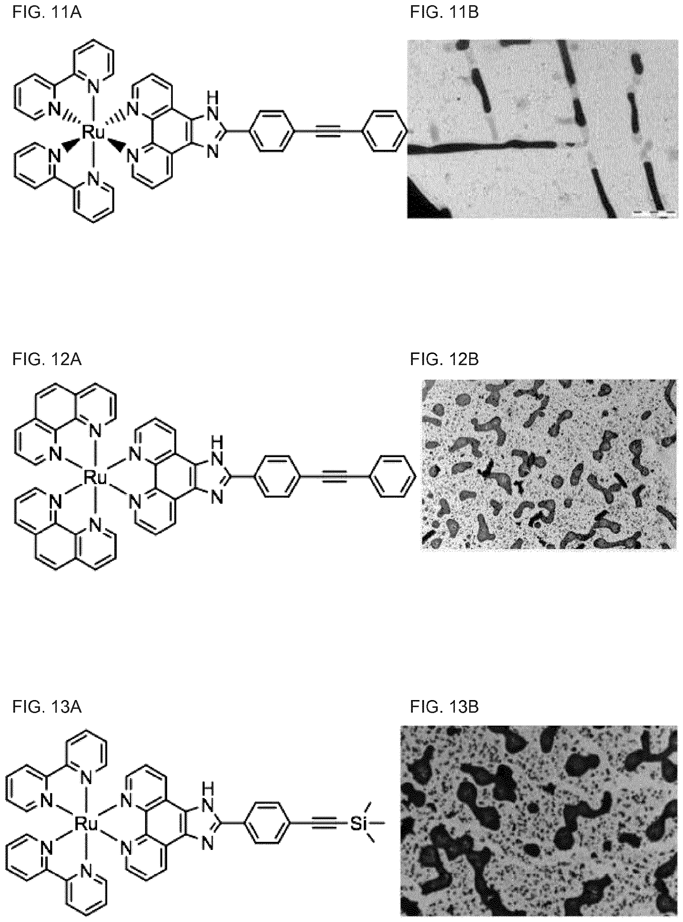

1 mM Δ-[Ru(bpy)₂pBEPIP](ClO₄)₂ (Fig.11A) is uniformly mixed with 1 mM c-myc Pu22 DNA solution in a ratio of 1:1, then the mixture obtained is heated to 90°C and maintained at the temperature for 0.5 to 10 min, then the mixture is naturally cooled down to room temperature and left to stand for 24 h at 4°C. The reaction mixture is dialyzed against distilled water to remove the polypyridyl ruthenium (II) complexes unloaded onto the nucleic acid, thereby to form a polypyridyl ruthenium (II) coordination complex - nucleic acid complex. It has been found by a TEM detection that the ruthenium complex facilitates self-assembly of the telomere DNA sequence to form an analogous nanotube structure (Fig.11B).

### Example 12: Preparation of [Ru(phen)₂pBEPIP](ClO₄)₂-c-myc Promoter Region DNA Nano-complex

1 mM [Ru(phen)₂pBEPIP](ClO₄)₂ (Fig.12A) is uniformly mixed with 1 mM c-myc Pu22 DNA solution in a ratio of 1:1, then the mixture obtained is heated to 90°C and maintained at the temperature for 0.5 to 10 min, then the mixture is naturally cooled down to room temperature and left to stand for 24 h at 4°C. The reaction mixture is dialyzed against distilled water to remove the polypyridyl ruthenium (II) complexes unloaded onto the nucleic acid, thereby to form a polypyridyl ruthenium (II) coordination complex - nucleic acid complex. It has been found by a TEM detection that the ruthenium complex facilitates self-assembly of the telomere DNA sequence to form an analogous nanotube structure (Fig.12B).

### Example 13: Preparation of [Ru(bpy)₂pTEPIP](ClO₄)₂-c-myc Promoter Region DNA Nano-complex

1 mM [Ru(bpy)₂pTEPIP](ClO₄)₂ (Fig.13A) is uniformly mixed with 1 mM c-myc Pu22 DNA solution in a ratio of 1:1, then the mixture obtained is heated to 90°C under microwave radiation and maintained at the temperature for 0.5 to 10 min, then the mixture is naturally cooled down to room temperature and left to stand for 24 h at 4°C. The reaction mixture is dialyzed against distilled water to remove the polypyridyl ruthenium (II) complexes unloaded onto the nucleic acid, thereby to form a polypyridyl ruthenium (II) coordination complex - nucleic acid complex. It has been found by a TEM detection that the ruthenium complex facilitates self-assembly of the telomere DNA sequence to form an analogous nanotube structure (Fig.13B).

### Example 14: Preparation of [Ru(phen)₂pTEPIP](ClO₄)₂-c-myc Promoter Region DNA Nano-complex

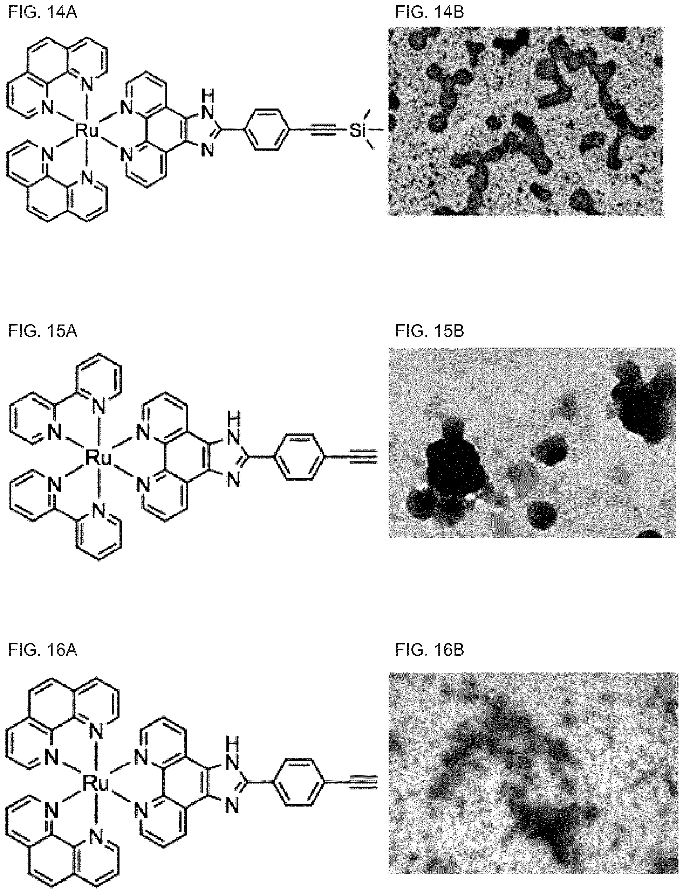

1 mM [Ru(phen)₂pTEPIP](ClO₄)₂ (Fig.14A) is uniformly mixed with 1 mM c-myc Pu22 DNA solution in a ratio of 1:1, then the mixture obtained is heated to 90°C under microwave radiation and maintained at the temperature for 0.5 to 10 min, then the mixture is naturally cooled down to room temperature and left to stand for 24 h at 4°C. The reaction mixture is dialyzed against distilled water to remove the polypyridyl ruthenium (II) complexes unloaded onto the nucleic acid, thereby to form a polypyridyl ruthenium (II) coordination complex - nucleic acid complex. It has been found by a TEM detection that the ruthenium complex facilitates self-assembly of the telomere DNA sequence to form an analogous nanotube structure (Fig.14B).

### Example 15: Preparation of [Ru(bpy)₂pEPIP](ClO₄)₂-c-myc Promoter Region DNA Nano-complex

1 mM [Ru(bpy)₂pEPIP](ClO₄)₂ (Fig.15A) is uniformly mixed with 1 mM c-myc Pu22 DNA solution in a ratio of 1:1, then the mixture obtained is heated to 90°C under microwave radiation and maintained at the temperature for 0.5 to 10 min, then the mixture is naturally cooled down to room temperature and left to stand for 24 h at 4°C. The reaction mixture is dialyzed against distilled water to remove the polypyridyl ruthenium (II) complexes unloaded onto the nucleic acid, thereby to form a polypyridyl ruthenium (II) coordination complex - nucleic acid complex. It has been found by a TEM detection that the ruthenium complex facilitates self-assembly of the telomere DNA sequence to form an analogous nanotube structure (Fig.15B).

### Example 16: Preparation of [Ru(phen)₂pEPIP](ClO₄)₂-c-myc Promoter Region DNA Nano-complex

1 mM [Ru(phen)₂pEPIP](ClO₄)₂ (Fig.16A) is uniformly mixed with 1 mM c-myc Pu22 DNA solution in a ratio of 1:1, then the mixture obtained is heated to 90°C under microwave radiation and maintained at the temperature for 0.5 to 10 min, then the mixture is naturally cooled down to room temperature and left to stand for 24 h at 4°C. The reaction mixture is dialyzed against distilled water to remove the polypyridyl ruthenium (II) complexes unloaded onto the nucleic acid, thereby to form a polypyridyl ruthenium (II) coordination complex - nucleic acid complex. It has been found by a TEM detection that the ruthenium complex facilitates self-assembly of the telomere DNA sequence to form an analogous nanotube structure (Fig.16B).

### Example 17: Preparation of [Ru(bpy)₂oBEPIP](ClO₄)₂-c-myc Promoter Region DNA Nano-complex

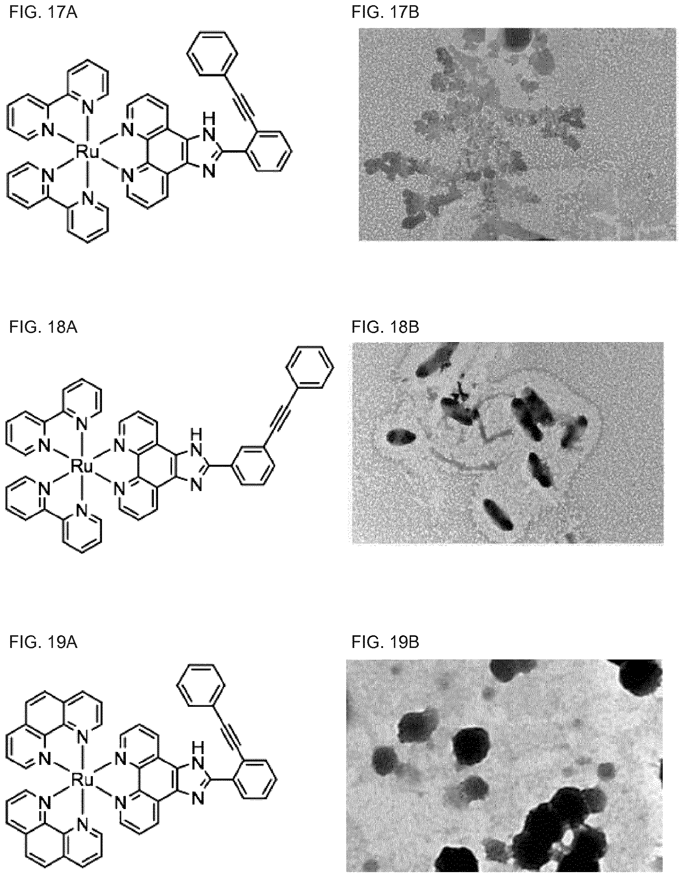

1 mM [Ru(bpy)₂oBEPIP](ClO₄)₂ (Fig.17A) is uniformly mixed with 1 mM c-myc Pu22 DNA solution in a ratio of 1:1, then the mixture obtained is heated to 90°C under microwave radiation and maintained at the temperature for 0.5 to 10 min, then the mixture is naturally cooled down to room temperature and left to stand for 24 h at 4°C. The reaction mixture is dialyzed against distilled water to remove the polypyridyl ruthenium (II) complexes unloaded onto the nucleic acid, thereby to form a polypyridyl ruthenium (II) coordination complex - nucleic acid complex. It has been found by a TEM detection that the ruthenium complex facilitates self-assembly of the telomere DNA sequence to form an analogous nanotube structure (Fig.17B).

### Example 18: Preparation of [Ru(bpy)₂mBEPIP](ClO₄)₂-c-myc Promoter Region DNA Nano-complex

1 mM [Ru(bpy)₂mBEPIP](ClO₄)₂ (Fig.18A) is uniformly mixed with 1 mM c-myc Pu22 DNA solution in a ratio of 1:1, then the mixture obtained is heated to 90°C under microwave radiation and maintained at the temperature for 0.5 to 10 min, then the mixture is naturally cooled down to room temperature and left to stand for 24 h at 4°C. The reaction mixture is dialyzed against distilled water to remove the polypyridyl ruthenium (II) complexes unloaded onto the nucleic acid, thereby to form a polypyridyl ruthenium (II) coordination complex - nucleic acid complex. It has been found by a TEM detection that the ruthenium complex facilitates self-assembly of the telomere DNA sequence to form an analogous nanotube structure (Fig.18B).

### Example 19: Preparation of [Ru(phen)₂oBEPIP](ClO₄)₂-c-myc Promoter Region DNA Nano-complex

1 mM [Ru(phen)₂oBEPIP](ClO₄)₂ (Fig.19A) is uniformly mixed with 1 mM c-myc Pu22 DNA solution in a ratio of 1:1, then the mixture obtained is heated to 90°C under microwave radiation and maintained at the temperature for 0.5 to 10 min, then the mixture is naturally cooled down to room temperature and left to stand for 24 h at 4°C. The reaction mixture is dialyzed against distilled water to remove the polypyridyl ruthenium (II) complexes unloaded onto the nucleic acid, thereby to form a polypyridyl ruthenium (II) coordination complex - nucleic acid complex. It has been found by a TEM detection that the ruthenium complex facilitates self-assembly of the telomere DNA sequence to form an analogous nanotube structure (Fig.19B).

### Example 20: Preparation of [Ru(phen)₂mBEPIP](ClO₄)₂-c-myc Promoter Region DNA Nano-complex

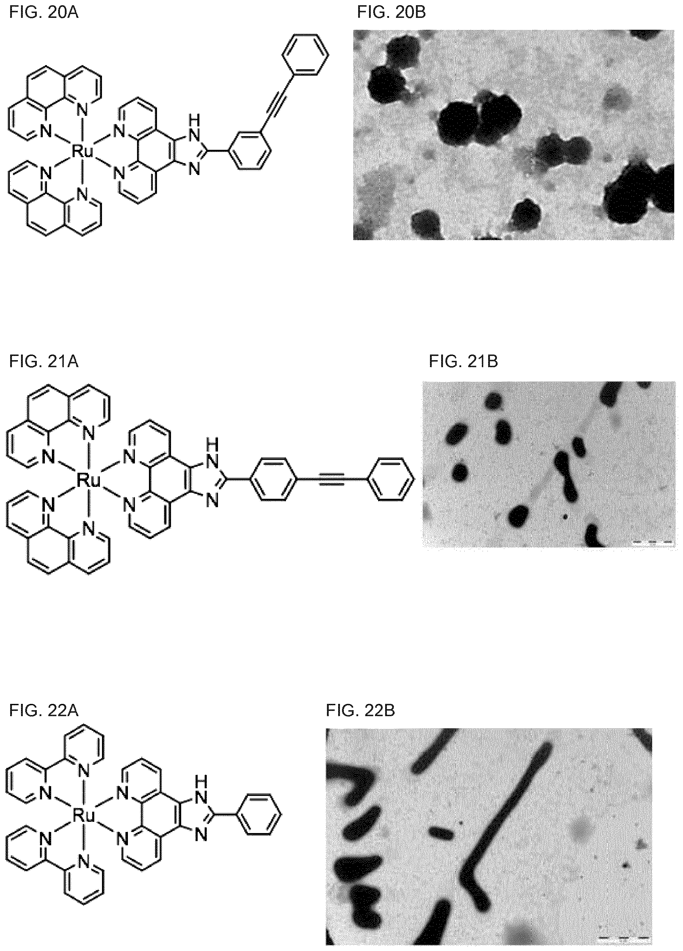

1 mM [Ru(phen)₂mBEPIP](ClO₄)₂ (Fig.20A) is uniformly mixed with 1 mM c-myc Pu22 DNA solution in a ratio of 1:1, then the mixture obtained is heated to 90°C under microwave radiation and maintained at the temperature for 0.5 to 10 min, then the mixture is naturally cooled down to room temperature and left to stand for 24 h at 4°C. The reaction mixture is dialyzed against distilled water to remove the polypyridyl ruthenium (II) complexes unloaded onto the nucleic acid, thereby to form a polypyridyl ruthenium (II) coordination complex - nucleic acid complex. It has been found by a TEM detection that the ruthenium complex facilitates self-assembly of the telomere DNA sequence to form an analogous nanotube structure (Fig.20B).

### Example 21: Preparation of [Ru(phen)₂pBEPIP](ClO₄)₂-c-myc Promoter Region DNA Nano-complex

1 mM [Ru(phen)₂pBEPIP](ClO₄)₂(Fig.21A) is uniformly mixed with 1 mM c-myc Pu22 DNA solution in a ratio of 1:1, then the mixture obtained is heated to 90°C under microwave radiation and maintained at the temperature for 0.5 to 10 min, then the mixture is naturally cooled down to room temperature and left to stand for 24 h at 4°C. The reaction mixture is dialyzed against distilled water to remove the polypyridyl ruthenium (II) complexes unloaded onto the nucleic acid, thereby to form a polypyridyl ruthenium (II) coordination complex - nucleic acid complex. It has been found by a TEM detection that the ruthenium complex facilitates self-assembly of the telomere DNA sequence to form an analogous nanotube structure (Fig.21B).

### Example 22: Preparation of [Ru(bpy)₂PIP](ClO₄)₂-c-myc Promoter Region DNA Nano-complex (Reference Example)

10 mM [Ru(bpy)₂PIP](ClO₄)₂ (Fig.22A) is uniformly mixed with 1 mM c-myc Pu22 DNA solution in a ratio of 10:1, then the mixture obtained is heated to 90°C under microwave radiation and maintained at the temperature for 0.5 to 10 min, then the mixture is naturally cooled down to room temperature and left to stand for 24 h at 4°C. The reaction mixture is dialyzed against distilled water to remove the polypyridyl ruthenium (II) complexes unloaded onto the nucleic acid, thereby to form a polypyridyl ruthenium (II) coordination complex - nucleic acid complex. It has been found by a TEM detection that the ruthenium complex facilitates self-assembly of the telomere DNA sequence to form an analogous nanotube structure (Fig.22B).

### Example 23: Preparation of [Ru(phen)₂PIP](ClO₄)₂-c-myc Promoter Region DNA Nano-complex (Reference Example)

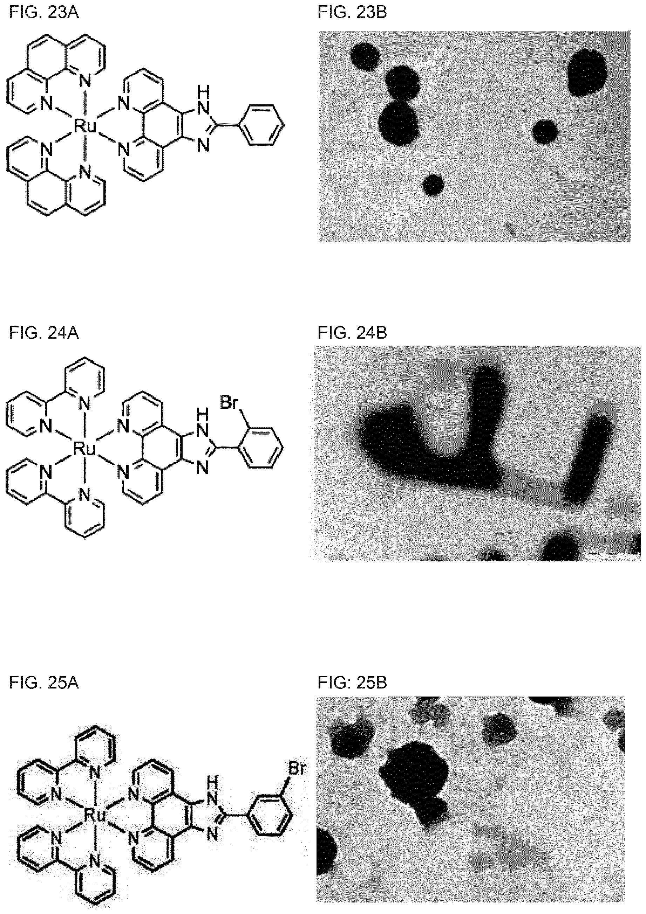

10 mM [Ru(phen)₂PIP](ClO₄)₂ (Fig.23A) is uniformly mixed with 1 mM c-myc Pu22 DNA solution in a ratio of 10:1, then the mixture obtained is heated to 90°C under microwave radiation and maintained at the temperature for 0.5 to 10 min, then the mixture is naturally cooled down to room temperature and left to stand for 24 h at 4°C. The reaction mixture is dialyzed against distilled water to remove the polypyridyl ruthenium (II) complexes unloaded onto the nucleic acid, thereby to form a polypyridyl ruthenium (II) coordination complex - nucleic acid complex. It has been found by a TEM detection that the ruthenium complex facilitates self-assembly of the telomere DNA sequence to form an analogous nanotube structure (Fig.23B).

### Example 24: Preparation of [Ru(bpy)₂oBrPIP](ClO₄)₂-c-myc Promoter Region DNA Nano-complex (Reference Example)

1 mM [Ru(bpy)₂oBrPIP](ClO₄)₂ (Fig.24A) is uniformly mixed with 10 mM c-myc Pu22 DNA solution in a ratio of 1:10, then the mixture obtained is heated to 90°C under microwave radiation and maintained at the temperature for 0.5 to 10 min, then the mixture is naturally cooled down to room temperature. The reaction mixture is dialyzed against distilled water to remove the polypyridyl ruthenium (II) complexes unloaded onto the nucleic acid, thereby to form a polypyridyl ruthenium (II) coordination complex - nucleic acid complex. It has been found by a TEM detection that the ruthenium complex facilitates self-assembly of the telomere DNA sequence to form an analogous nanotube structure (Fig.24B).

### Example 25: Preparation of [Ru(bpy)₂mBrPIP](ClO₄)₂-c-myc Promoter Region DNA Nano-complex (Reference Example)

1 mM [Ru(bpy)₂mBrPIP](ClO₄)₂ (Fig.25A) is uniformly mixed with 10 mM c-myc Pu22 DNA solution in a ratio of 1:10, then the mixture obtained is heated to 90°C under microwave radiation and maintained at the temperature for 0.5 to 10 min, then the mixture is naturally cooled down to room temperature. The reaction mixture is dialyzed against distilled water to remove the polypyridyl ruthenium (II) complexes unloaded onto the nucleic acid, thereby to form a polypyridyl ruthenium (II) coordination complex - nucleic acid complex. It has been found by a TEM detection that the ruthenium complex facilitates self-assembly of the telomere DNA sequence to form an analogous nanotube structure (Fig.25B).

### Example 26: Preparation of [Ru(bpy)₂pBrPIP](ClO₄)₂-c-myc Promoter Region DNA Nano-complex (Reference Example)

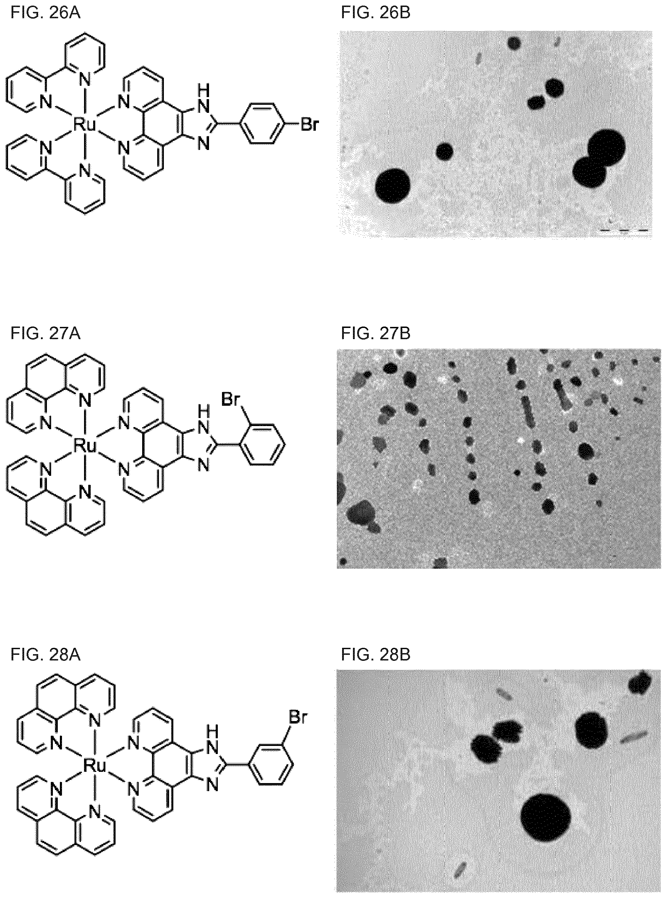

1 mM [Ru(bpy)₂pBrPIP](ClO₄)₂ (Fig.26A) is uniformly mixed with 1 mM c-myc Pu22 DNA solution in a ratio of 1:1, then the mixture obtained is heated to 90°C under microwave radiation and maintained at the temperature for 0.5 to 10 min, then the mixture is naturally cooled down to room temperature and left to stand for 24 h at 4°C. The reaction mixture is dialyzed against distilled water to remove the polypyridyl ruthenium (II) complexes unloaded onto the nucleic acid, thereby to form a polypyridyl ruthenium (II) coordination complex - nucleic acid complex. It has been found by a TEM detection that the ruthenium complex facilitates self-assembly of the telomere DNA sequence to form an analogous nanotube structure (Fig.26B).

### Example 27: Preparation of [Ru(phen)₂oBrPIP](ClO₄)₂-c-myc Promoter Region DNA Nano-complex (Reference Example)

1 mM [Ru(phen)₂oBrPIP](ClO₄)₂ is uniformly mixed with 1 mM c-myc Pu22 DNA solution in a ratio of 1:1, then the mixture obtained is heated to 90°C under microwave radiation and maintained at the temperature for 0.5 to 10 min, then the mixture is naturally cooled down to room temperature and left to stand for 24 h at 4°C. The reaction mixture is dialyzed against distilled water to remove the polypyridyl ruthenium (II) complexes unloaded onto the nucleic acid, thereby to form a polypyridyl ruthenium (II) coordination complex - nucleic acid complex. It has been found by a TEM detection that the ruthenium complex facilitates self-assembly of the telomere DNA sequence to form an analogous nanotube structure (Fig.27).

### Example 28: Preparation of [Ru(phen)₂mBrPIP](ClO₄)₂-c-myc Promoter Region DNA Nano-complex (Reference Example)

1 mM [Ru(phen)₂mBrPIP](ClO₄)₂ is uniformly mixed with 1 mM c-myc Pu22 DNA solution in a ratio of 1:1, then the mixture obtained is heated to 90°C under microwave radiation and maintained at the temperature for 0.5 to 10 min, then the mixture is naturally cooled down to room temperature and left to stand for 24 h at 4°C. The reaction mixture is dialyzed against distilled water to remove the polypyridyl ruthenium (II) complexes unloaded onto the nucleic acid, thereby to form a polypyridyl ruthenium (II) coordination complex - nucleic acid complex. It has been found by a TEM detection that the ruthenium complex facilitates self-assembly of the telomere DNA sequence to form an analogous nanotube structure (Fig.28).

### Example 29: Preparation of [Ru(phen)₂pBrPIP](ClO₄)₂-c-myc Promoter Region DNA Nano-complex (Reference Example)

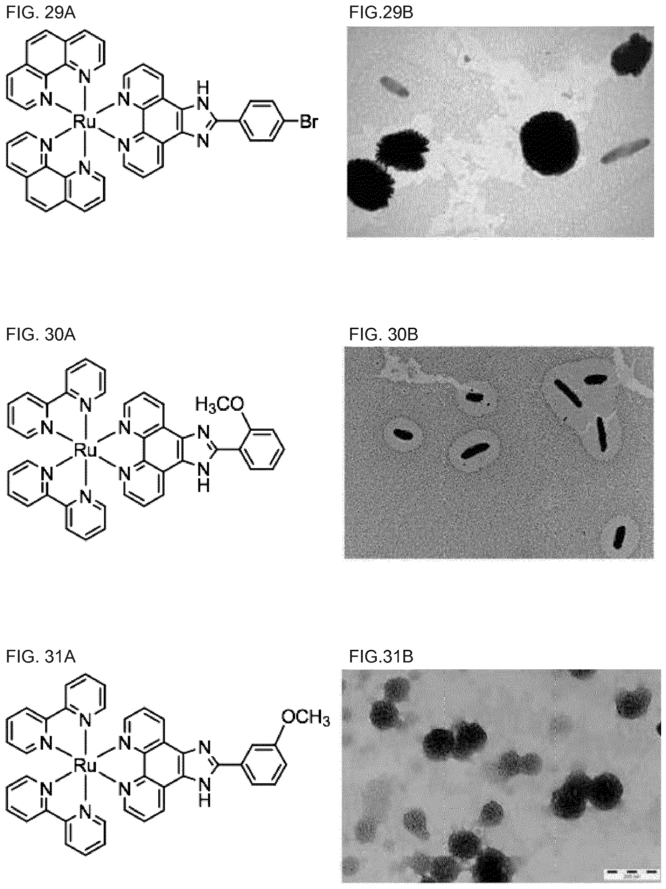

1 mM [Ru(phen)₂pBrPIP](ClO₄)₂ is uniformly mixed with 1 mM c-myc Pu22 DNA solution in a ratio of 1:1, then the mixture obtained is heated to 90°C under microwave radiation and maintained at the temperature for 0.5 to 10 min, then the mixture is naturally cooled down to room temperature and left to stand for 24 h at 4°C. The reaction mixture is dialyzed against distilled water to remove the polypyridyl ruthenium (II) complexes unloaded onto the nucleic acid, thereby to form a polypyridyl ruthenium (II) coordination complex - nucleic acid complex. It has been found by a TEM detection that the ruthenium complex facilitates self-assembly of the telomere DNA sequence to form an analogous nanotube structure (Fig.29).

### Example 30: Preparation of [Ru(bpy)₂oMOPIP](ClO₄)₂-c-myc Promoter Region DNA Nano-complex (Reference Example)

1 mM [Ru(bpy)₂oMOPIP](ClO₄)₂ is uniformly mixed with 1 mM c-myc Pu22 DNA solution in a ratio of 1:1, then the mixture obtained is heated to 90°C under microwave radiation and maintained at the temperature for 0.5 to 10 min, then the mixture is naturally cooled down to room temperature and left to stand for 24 h at 4°C. The reaction mixture is dialyzed against distilled water to remove the polypyridyl ruthenium (II) complexes unloaded onto the nucleic acid, thereby to form a polypyridyl ruthenium (II) coordination complex - nucleic acid complex. It has been found by a TEM detection that the ruthenium complex facilitates self-assembly of the telomere DNA sequence to form an analogous nanotube structure (Fig.30).

### Example 31: Preparation of [Ru(bpy)₂mMOPIP](ClO₄)₂-c-myc Promoter Region DNA Nano-complex (Reference Example)

1 mM [Ru(bpy)₂mMOPIP](ClO₄)₂ is uniformly mixed with 1 mM c-myc Pu22 DNA solution in a ratio of 1:1, then the mixture obtained is heated to 90°C under microwave radiation and maintained at the temperature for 0.5 to 10 min, then the mixture is naturally cooled down to room temperature and left to stand for 24 h at 4°C. The reaction mixture is dialyzed against distilled water to remove the polypyridyl ruthenium (II) complexes unloaded onto the nucleic acid, thereby to form a polypyridyl ruthenium (II) coordination complex - nucleic acid complex. It has been found by a TEM detection that the ruthenium complex facilitates self-assembly of the telomere DNA sequence to form an analogous nanotube structure (Fig.31).

### Example 32: Preparation of [Ru(bpy)₂pMOPIP](ClO₄)₂-c-myc Promoter Region DNA Nano-complex (Reference Example)

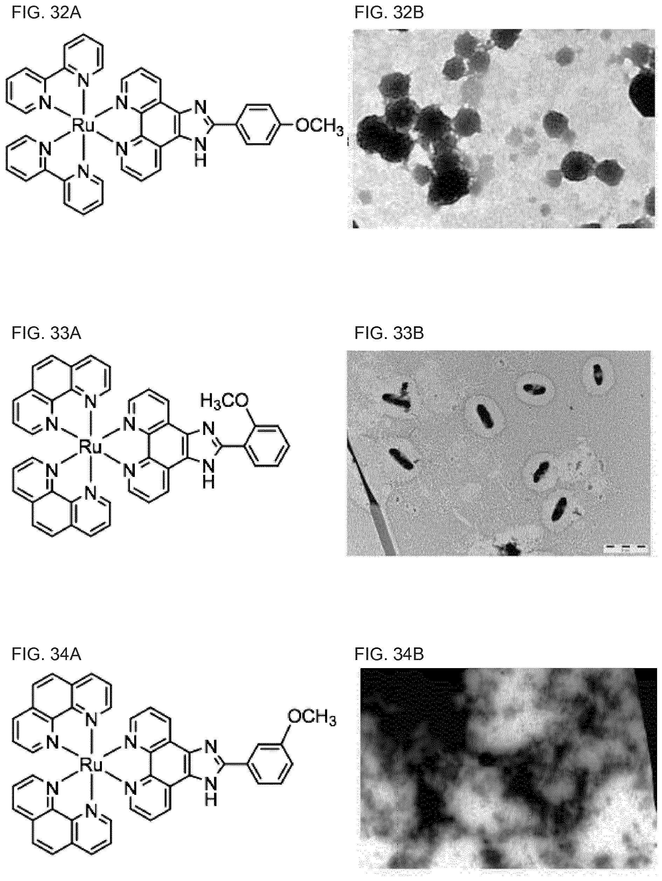

1 mM [Ru(bpy)₂pMOPIP](ClO₄)₂ is uniformly mixed with 1 mM c-myc Pu22 DNA solution in a ratio of 1:1, then the mixture obtained is heated to 90°C under microwave radiation and maintained at the temperature for 0.5 to 10 min, then the mixture is naturally cooled down to room temperature and left to stand for 24 h at 4°C. The reaction mixture is dialyzed against distilled water to remove the polypyridyl ruthenium (II) complexes unloaded onto the nucleic acid, thereby to form a polypyridyl ruthenium (II) coordination complex - nucleic acid complex. It has been found by a TEM detection that the ruthenium complex facilitates self-assembly of the telomere DNA sequence to form an analogous nanotube structure (Fig.32).

### Example 33: Preparation of [Ru(phen)₂oMOPIP](ClO₄)₂-c-myc Promoter Region DNA Nano-complex (Reference Example)

1 mM [Ru(phen)₂oMOPIP](ClO₄)₂ (Fig.33A) is uniformly mixed with 1 mM c-myc Pu22 DNA solution in a ratio of 1:1, then the mixture obtained is heated to 90°C under microwave radiation and maintained at the temperature for 0.5 to 10 min, then the mixture is naturally cooled down to room temperature and left to stand for 24 h at 4°C. The reaction mixture is dialyzed against distilled water to remove the polypyridyl ruthenium (II) complexes unloaded onto the nucleic acid, thereby to form a polypyridyl ruthenium (II) coordination complex - nucleic acid complex. It has been found by a TEM detection that the ruthenium complex facilitates self-assembly of the telomere DNA sequence to form an analogous nanotube structure (Fig.33B).

### Example 34: Preparation of [Ru(phen)₂mMOPIP](ClO₄)₂-c-myc Promoter Region DNA Nano-complex (Reference Example)

1 mM [Ru(phen)₂mMOPIP](ClO₄)₂ (Fig.34A) is uniformly mixed with 1 mM c-myc Pu22 DNA solution in a ratio of 1:1, then the mixture obtained is heated to 90°C under microwave radiation and maintained at the temperature for 0.5 to 10 min, then the mixture is naturally cooled down to room temperature and left to stand for 24 h at 4°C. The reaction mixture is dialyzed against distilled water to remove the polypyridyl ruthenium (II) complexes unloaded onto the nucleic acid, thereby to form a polypyridyl ruthenium (II) coordination complex - nucleic acid complex. It has been found by a TEM detection that the ruthenium complex facilitates self-assembly of the telomere DNA sequence to form an analogous nanotube structure (Fig.34B).

### Example 35: Preparation of [Ru(phen)₂pMOPIP](ClO₄)₂-c-myc Promoter Region DNA Nano-complex (Reference Example)

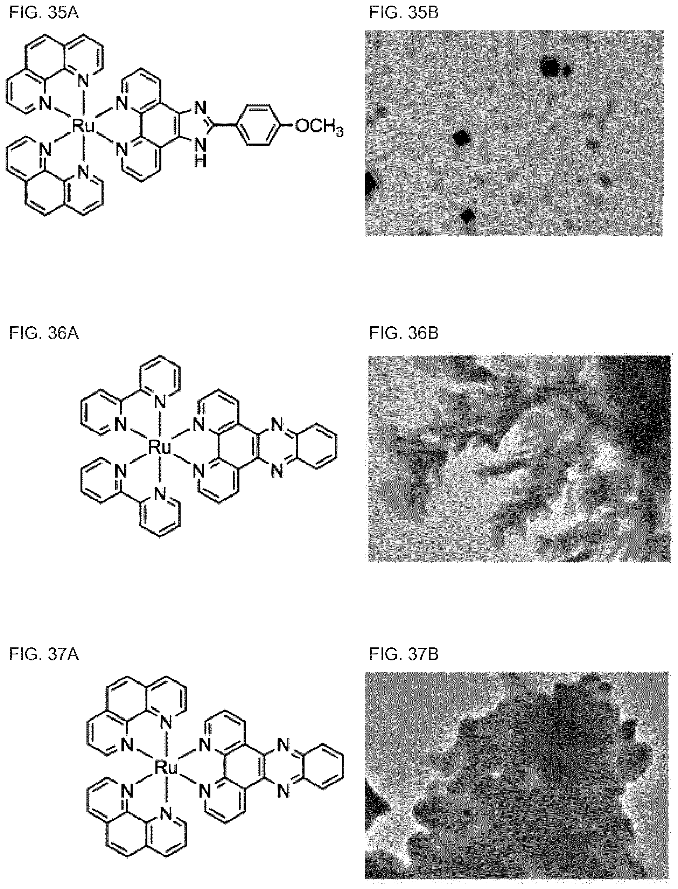

1 mM [Ru(phen)₂pMOPIP](ClO₄)₂ (Fig.35A) is uniformly mixed with 1 mM c-myc Pu22 DNA solution in a ratio of 1:1, then the mixture obtained is heated to 90°C under microwave radiation and maintained at the temperature for 0.5 to 10 min, then the mixture is naturally cooled down to room temperature and left to stand for 24 h at 4°C. The reaction mixture is dialyzed against distilled water to remove the polypyridyl ruthenium (II) complexes unloaded onto the nucleic acid, thereby to form a polypyridyl ruthenium (II) coordination complex - nucleic acid complex. It has been found by a TEM detection that the ruthenium complex facilitates self-assembly of the telomere DNA sequence to form an analogous nanotube structure (Fig.35B).

### Example 36: Preparation of [Ru(bpy)₂DPPZ](ClO₄)₂-c-myc Promoter Region DNA Nano-complex (Reference Example)

1 mM [Ru(bpy)₂DPPZ](ClO₄)₂ (Fig.36A) is uniformly mixed with 1 mM c-myc Pu22 DNA solution in a ratio of 1:1, then the mixture obtained is heated to 90°C under microwave radiation and maintained at the temperature for 0.5 to 10 min, then the mixture is naturally cooled down to room temperature and left to stand for 24 h at 4°C. The reaction mixture is dialyzed against distilled water to remove the polypyridyl ruthenium (II) complexes unloaded onto the nucleic acid, thereby to form a polypyridyl ruthenium (II) coordination complex - nucleic acid complex. It has been found by a TEM detection that the ruthenium complex facilitates self-assembly of the telomere DNA sequence to form an analogous nanotube structure (Fig.36B).

### Example 37: Preparation of [Ru(phen)₂DPPZ](ClO₄)₂-c-myc Promoter Region DNA Nano-complex (Reference Example)

1 mM [Ru(phen)₂DPPZ](ClO₄)₂ (Fig.37A) is uniformly mixed with 1 mM c-myc Pu22 DNA solution in a ratio of 1:1, then the mixture obtained is heated to 90°C under microwave radiation and maintained at the temperature for 0.5 to 10 min, then the mixture is naturally cooled down to room temperature and left to stand for 24 h at 4°C. The reaction mixture is dialyzed against distilled water to remove the polypyridyl ruthenium (II) complexes unloaded onto the nucleic acid, thereby to form a polypyridyl ruthenium (II) coordination complex - nucleic acid complex. It has been found by a TEM detection that the ruthenium complex facilitates self-assembly of the telomere DNA sequence to form an analogous nanotube structure (Fig.37B).

### Example 38: Preparation of [Ru(bpy)₂3-BrDPPZ](ClO₄)₂-c-myc Promoter Region DNA Nano-complex (Reference Example)

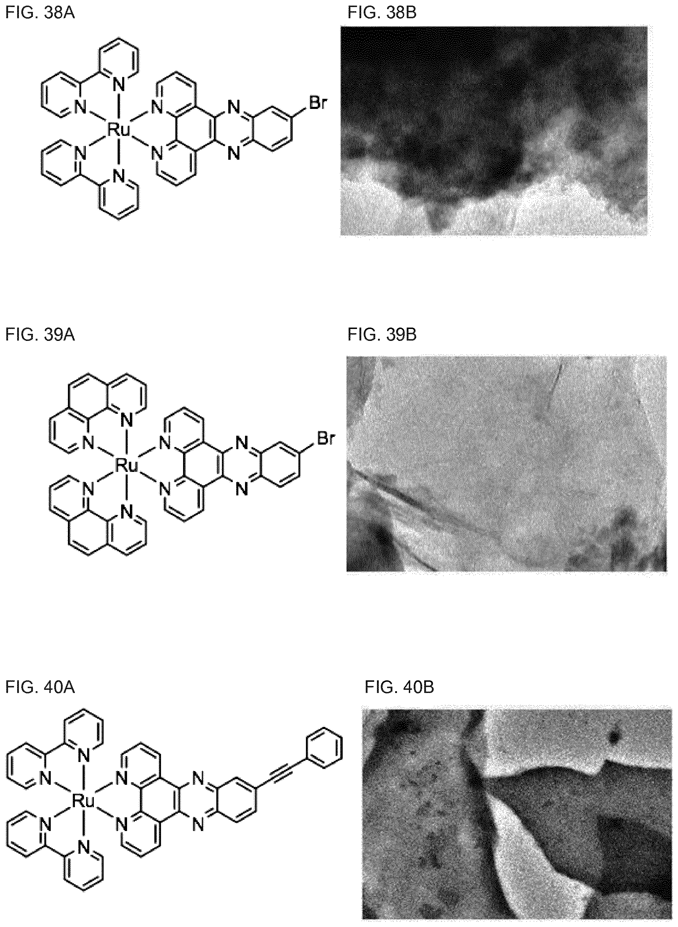

1 mM [Ru(bpy)₂3-BrDPPZ](ClO₄)₂ (Fig.38A) is uniformly mixed with 1 mM c-myc Pu22 DNA solution in a ratio of 1:1, then the mixture obtained is heated to 90°C under microwave radiation and maintained at the temperature for 0.5 to 10 min, then the mixture is naturally cooled down to room temperature and left to stand for 24 h at 4°C. The reaction mixture is dialyzed against distilled water to remove the polypyridyl ruthenium (II) complexes unloaded onto the nucleic acid, thereby to form a polypyridyl ruthenium (II) coordination complex - nucleic acid complex. It has been found by a TEM detection that the ruthenium complex facilitates self-assembly of the telomere DNA sequence to form an analogous nanotube structure (Fig.38B).

### Example 39: Preparation of [Ru(phen)₂3-BrDPPZ](ClO₄)₂-c-myc Promoter Region DNA Nano-complex (Reference Example)

1 mM [Ru(phen)₂3-BrDPPZ](ClO₄)₂ (Fig.39A) is uniformly mixed with 1 mM c-myc Pu22 DNA solution in a ratio of 1:1, then the mixture obtained is heated to 90°C under microwave radiation and maintained at the temperature for 0.5 to 10 min, then the mixture is naturally cooled down to room temperature and left to stand for 24 h at 4°C. The reaction mixture is dialyzed against distilled water to remove the polypyridyl ruthenium (II) complexes unloaded onto the nucleic acid, thereby to form a polypyridyl ruthenium (II) coordination complex - nucleic acid complex. It has been found by a TEM detection that the ruthenium complex facilitates self-assembly of the telomere DNA sequence to form an analogous nanotube structure (Fig.39B).

### Example 40: Preparation of [Ru(bpy)₂3-BEDPPZ](ClO₄)₂-c-myc Promoter Region DNA Nano-complex

1 mM [Ru(bpy)₂3-BEDPPZ](ClO₄)₂ (Fig.40A) is uniformly mixed with 1 mM c-myc Pu22 DNA solution in a ratio of 1:1, then the mixture obtained is heated to 90°C under microwave radiation and maintained at the temperature for 0.5 to 10 min, then the mixture is naturally cooled down to room temperature and left to stand for 24 h at 4°C. The reaction mixture is dialyzed against distilled water to remove the polypyridyl ruthenium (II) complexes unloaded onto the nucleic acid, thereby to form a polypyridyl ruthenium (II) coordination complex - nucleic acid complex. It has been found by a TEM detection that the ruthenium complex facilitates self-assembly of the telomere DNA sequence to form an analogous nanotube structure (Fig.40B).

### Example 41: Preparation of [Ru(phen)₂3-BEDPPZ](ClO₄)₂-c-myc Promoter Region DNA Nano-complex

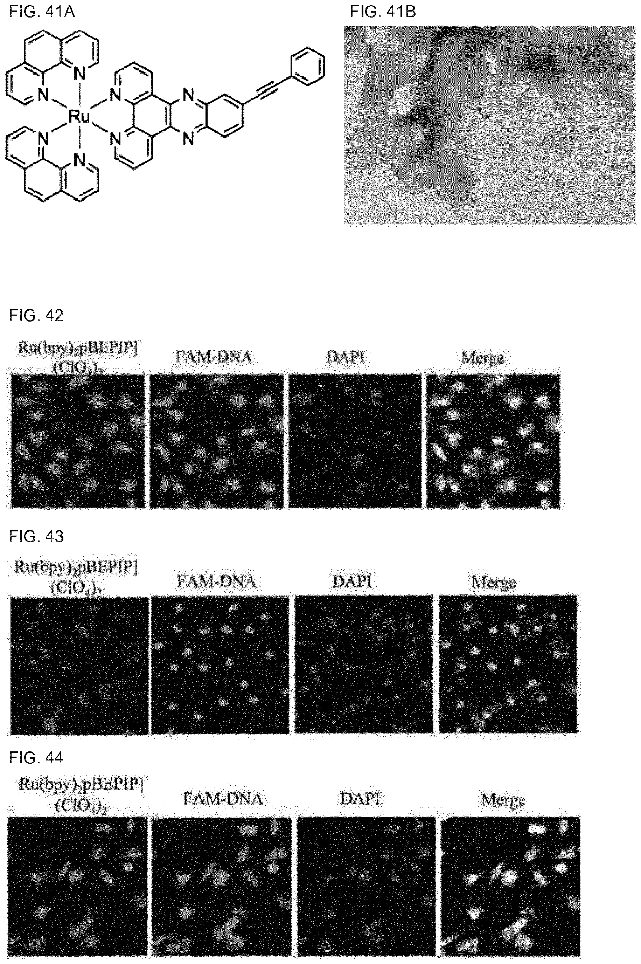

1 mM [Ru(phen)₂3-BEDPPZ](ClO₄)₂ (Fig.41A) is uniformly mixed with 1 mM c-myc Pu22 DNA solution in a ratio of 1:1, then the mixture obtained is heated to 90°C under microwave radiation and maintained at the temperature for 0.5 to 10 min, then the mixture is naturally cooled down to room temperature and left to stand for 24 h at 4°C. The reaction mixture is dialyzed against distilled water to remove the polypyridyl ruthenium (II) complexes unloaded onto the nucleic acid, thereby to form a polypyridyl ruthenium (II) coordination complex - nucleic acid complex. It has been found by a TEM detection that the ruthenium complex facilitates self-assembly of the telomere DNA sequence to form an analogous nanotube structure (Fig.41B).

### Absorption and Distribution of the Nano-complex Formed from Racemic Alkynyl Ruthenium Coordination Complex Ru(bpy)₂pBEPIP](ClO₄)₂ and c-myc DNA (complex of Example 1) for HepG2 Cells

Cells in the logarithmic growth phase are seeded in a 2 cm cell culture vessel at a density of 2×10⁵ cells per well. 1 mL DMEM comprising 5 µM ruthenium coordination complex - nucleic acid (FAM fluorescence-labeled) complex (Ru(bpy)₂pBEPIP](ClO₄)₂: FAM-c-myc pu22 DNA=1:1) is added and incubated in a 5% CO₂ incubator at 37°C for 2 h, then the incubated cells are observed under a fluorescence microscope and the results are shown in Fig.42. As can be seen from the figure, nucleic acids can be transferred into viable cells by the chiral alkynyl ruthenium complexes and distributed in the whole cells.

### Absorption and Distribution of the Nano-complex Formed from Racemic Alkynyl Ruthenium Coordination Complex Ru(bpy)₂pBEPIP](ClO₄)₂ and c-myc DNA (complex of Example 1) for Hela Cells.

Cells in the logarithmic growth phase are seeded in a 2 cm cell culture vessel at a density of 2×10⁵ cells per well. 1 mL DMEM comprising 5 µM ruthenium coordination complex - nucleic acid (FAM fluorescence-labeled) complex (Ru(bpy)₂pBEPIP](ClO₄)₂: FAM-c-myc pu22 DNA=1:1) is added and incubated in a 5% CO₂ incubator at 37°C for 2 h, then the incubated cells are observed under a fluorescence microscope and the results are shown in Fig.43. As can be seen from the figure, nucleic acids can be transferred into viable cells by the racemic alkynyl ruthenium complexes and distributed in the whole cells.

### Absorption and Distribution of the Nano-complex Formed from Racemic Alkynyl Ruthenium Coordination Complex Ru(bpy)₂pBEPIP](ClO₄)₂ and c-myc DNA (complex of Example 1) for MCF-7 Cells

Cells in the logarithmic growth phase are seeded in a 2 cm cell culture vessel at a density of 2×10⁵ cells per well. 1 mL DMEM comprising 5 µM ruthenium coordination complex - nucleic acid (FAM fluorescence-labeled) complex (Ru(bpy)₂pBEPIP](ClO₄)₂: FAM-c-myc pu22 DNA=1:1) is added and incubated in a 5% CO₂ incubator at 37°C for 2 h, then the incubated cells are observed under a fluorescence microscope and the results are shown in Fig.44. As can be seen from the figure, nucleic acids can be transferred into a viable cell by the racemic alkynyl ruthenium complexes and distributed in the whole cell.

### Absorption and Distribution of the Nano-complex Formed from L-alkynyl Ruthenium Coordination Complex [Λ-Ru(bpy)₂pBEPIP](ClO₄)₂ and c-myc DNA (complex of Example 10) for HepG2 Cells

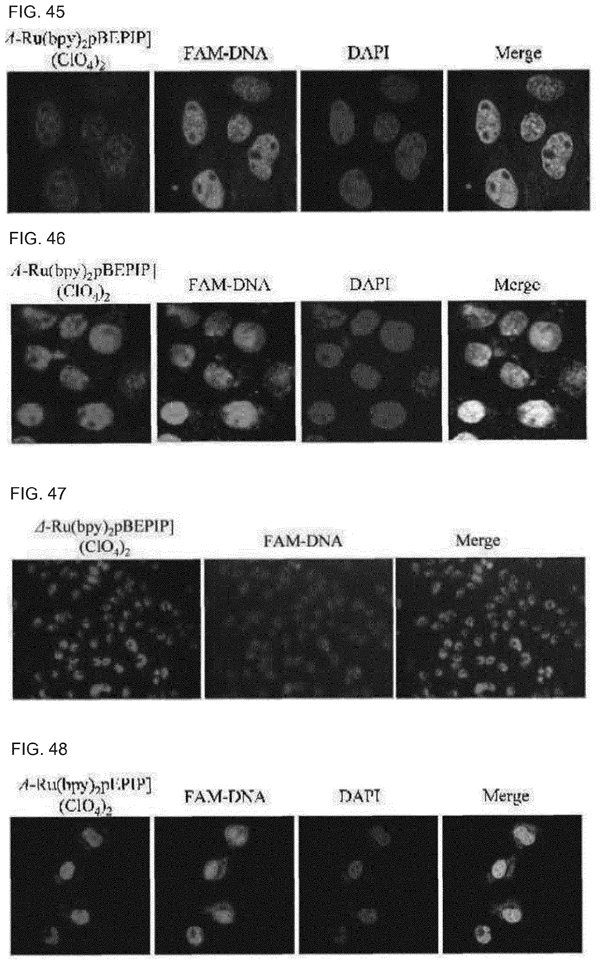

Cells in the logarithmic growth phase are seeded in a 2 cm cell culture vessel at a density of 2×10⁵ cells per well. 1 mL DMEM comprising 5 µM ruthenium coordination complex - nucleic acid (FAM fluorescence-labeled) complex [Λ-Ru(bpy)₂pBEPIP] (ClO₄)₂: FAM-c-myc pu22 DNA=1:1) is added and incubated in a 5% CO₂ incubator at 37°C for 2 h, then the incubated cells are observed under a fluorescence microscope and the results are shown in Fig.45. As can be seen from the figure, nucleic acids can be transferred into a viable cell by the L-alkynyl ruthenium complexes and distributed in the cell nucleus.

### Absorption and Distribution of the Nano-complex Formed from L-alkynyl Ruthenium Coordination Complex [Λ-Ru(bpy)₂pBEPIP](ClO₄)₂ and c-myc DNA (complex of Example 10) for MCF-7 Cells

Cells in the logarithmic growth phase are seeded in a 2 cm cell culture vessel at a density of 2×10⁵ cells per well. 1 mL DMEM comprising 5 µM ruthenium coordination complex - nucleic acid (FAM fluorescence-labeled) complex [Λ-Ru(bpy)₂pBEPIP] (ClO₄)₂: FAM-c-myc pu22 DNA=1:1) is added and incubated in a 5% CO₂ incubator at 37°C for 2 h, then the incubated cells are observed under a fluorescence microscope and the results are shown in Fig.46. As can be seen from the figure, nucleic acids can be transferred into a viable cell by the L-alkynyl ruthenium complexes and distributed in the cell nucleus.

### Absorption and Distribution of the Nano-complex Formed from R-alkynyl Ruthenium Coordination Complex [Δ-Ru(bpy)₂pBEPIP](ClO₄)₂ and c-myc DNA (complex of Example 11) for HepG2 Cells

Cells in the logarithmic growth phase are seeded in a 2 cm cell culture vessel at a density of 2×10⁵ cells per well. 1 mL DMEM comprising 5 µM ruthenium coordination complex - nucleic acid (FAM fluorescence-labeled) complex [Δ-Ru(bpy)₂pBEPIP] (ClO₄)₂: FAM-c-myc pu22 DNA=1:1) is added and incubated in a 5% CO₂ incubator at 37°C for 2 h, then the incubated cells are observed under a fluorescence microscope and the results are shown in Fig.47. As can be seen from the figure, nucleic acids can be transferred into a viable cell by the R-alkynyl ruthenium complexes and distributed in the cytoplasm.

### Absorption and Distribution of the Nano-complex Formed from L-alkynyl Ruthenium Coordination Complex [Λ-Ru(bpy)₂pBEPIP](ClO₄)₂ and AS1411 DNA (complex of Example 11) for MCF-7 Cells

Cells in the logarithmic growth phase are seeded in a 2 cm cell culture vessel at a density of 2×10⁵ cells per well. 1 mL DMEM comprising 5 µM ruthenium coordination complex - nucleic acid (FAM fluorescence-labeled) complex [Δ-Ru(bpy)₂pBEPIP](ClO₄)₂: FAM-AS1411 DNA=1:1) is added and incubated in a 5% CO₂ incubator at 37°C for 2 h, then the incubated cells are observed under a fluorescence microscope and the results are shown in Fig.48. As can be seen from the figure, nucleic acids can be transferred into a viable cell by the R-alkynyl ruthenium complexes and distributed in the cell nucleus.

## Claims

1. A non-therapeutic application of ruthenium-nucleic acid complexes, wherein the ruthenium complex has one of the following general formula: 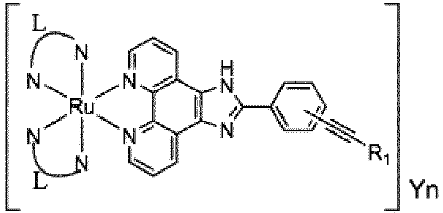 or 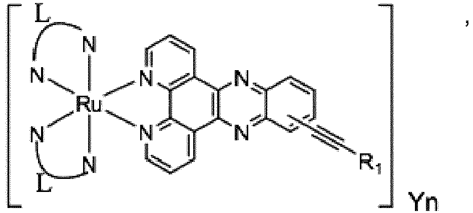 wherein:
**L** is an auxiliary ligand having a structural formula of: 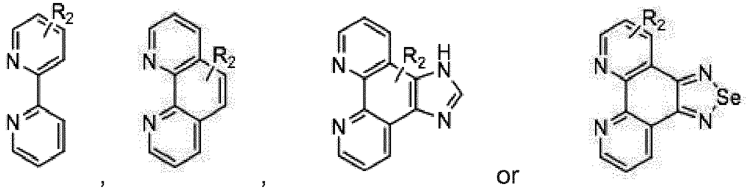
**R₁** is independently selected from H, hydroxyl, trimethylsilyl, C₁-C₆ alkyl or substituted C₁-C₆ alkyl, phenyl or substituted phenyl, pyridyl or substituted pyridyl, furyl or substituted furyl, pyrryl or substituted pyrryl, thiazolyl or substituted thiazolyl; wherein the position of R₁ substituted ethynyl may be ortho-position, meta-position or para-position on the benzene ring; the number of substituted ethynyl is 1, 2 or more;
**R₂** is independently selected from H, methyl, methoxyl, nitro and halogen;
**Y** is an ion or acidic radical ion which makes the whole ruthenium(II) complex be electrically neutral,
**n** is the number of ions or acidic radical ions which makes the whole ruthenium(II) complex be electrically neutral; and the length of the nucleic acid sequence is at least 4 bp,
and wherein the nucleic acid is selected from c-myc promoter region DNA, C-kit promoter region DNA, bcl-2 promoter region DNA, miR-21, DNA of AS1411, siRNA, microRNA, aptamer and mRNA;
wherein the nucleic acid is a fluorophore-labeled nucleic acid;
wherein the ruthenium complexes are employed as nucleic acid vectors, and wherein nucleic acid sequences are delivered into cells for fluorescent tracking.

2. The non-therapeutic application of ruthenium-nucleic acid complexes according to claim 1, wherein the ruthenium complex is selected from:

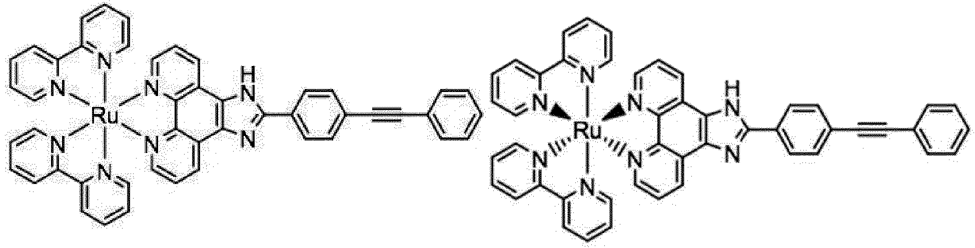

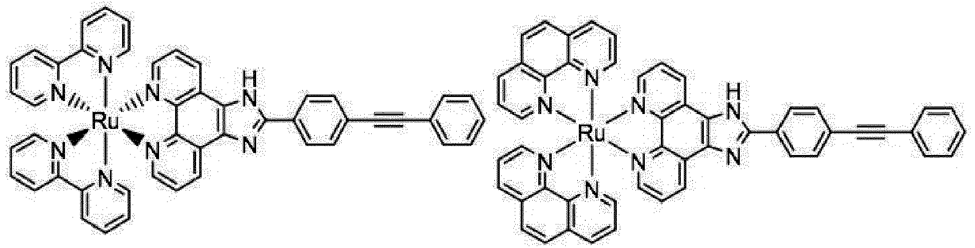

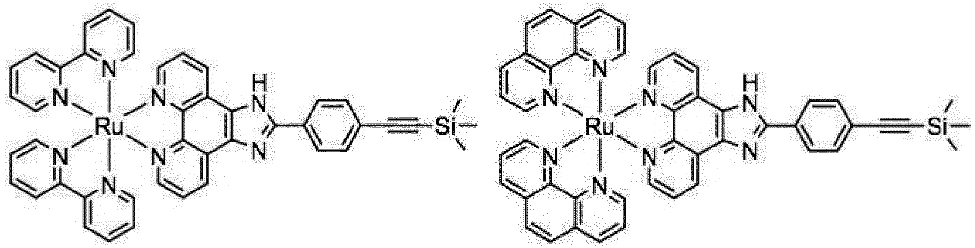

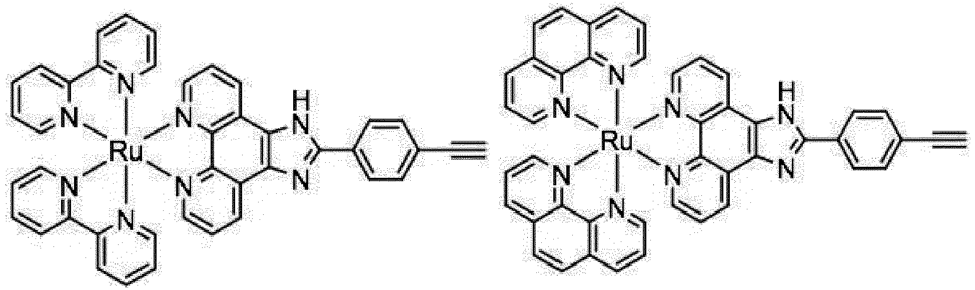

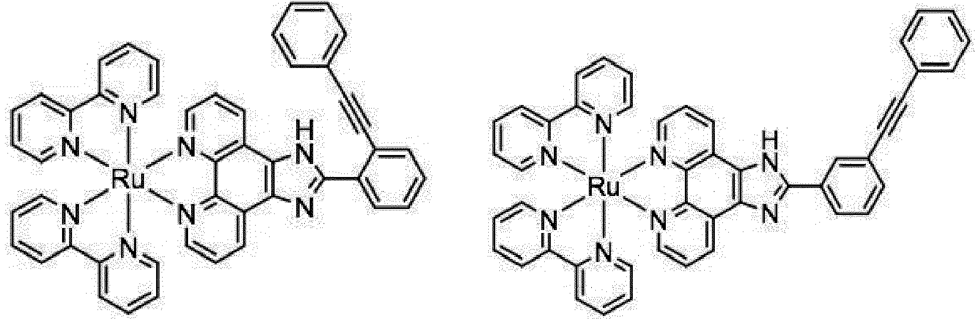

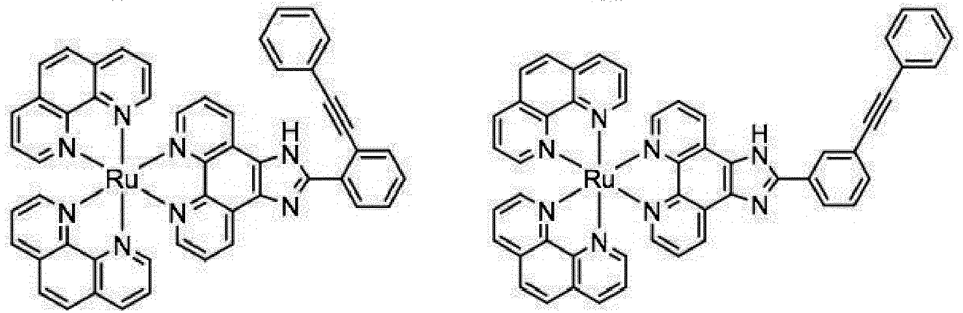

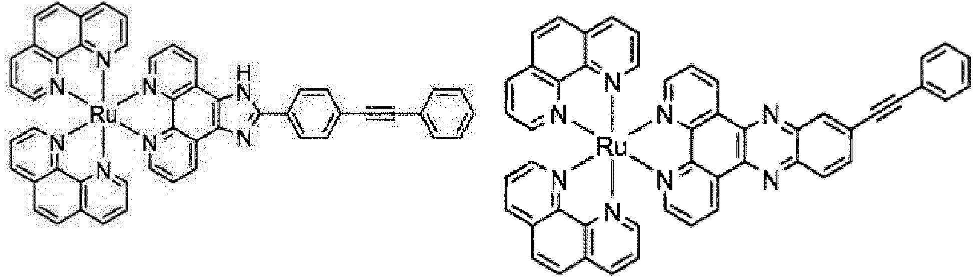

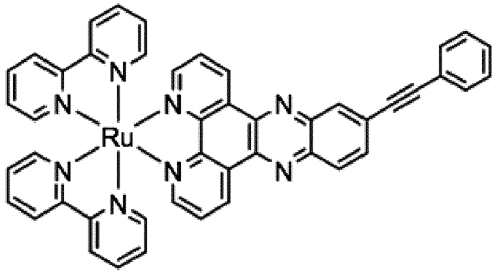

3. The non-therapeutic application of ruthenium-nucleic acid complexes according to claim 1 or 2, which is **characterized in that** the ruthenium complex is a single chiral isomer thereof.

4. The non-therapeutic application of ruthenium-nucleic acid complexes according to claim 1, which is **characterized in that** the length of the nucleic acid is not longer than 3,000 bp.

5. A method of preparing ruthenium coordination complex-nucleic acid complex, comprising the following steps:
the ruthenium complex is uniformly mixed with the nucleic acid solution, then the mixture obtained is heated to a temperature ranged from 70 to 100 °C and maintained at the temperature for 30 s to 30 min;
free ruthenium complexes are removed after cooling to form a ruthenium coordination complex-nucleic acid complex;
wherein the ruthenium complex has a structural formula according to any of claims 1-3 and the length of the nucleic acid is not shorter than 4 bp, and wherein the nucleic acid is selected from c-myc promoter region DNA, C-kit promoter region DNA, bcl-2 promoter region DNA, miR-21, DNA of AS1411, siRNA, microRNA, aptamer and mRNA;
wherein the nucleic acid is a fluorophore-labeled nucleic acid.

6. The method according to claim 5, which is **characterized in that** the heating is performed by a microwave.

## Patentansprüche

1. Nichttherapeutische Anwendung von Ruthenium-Nukleinsäure-Komplexen, wobei der Ruthenium-Komplex eine der folgenden allgemeinen Formeln aufweist: 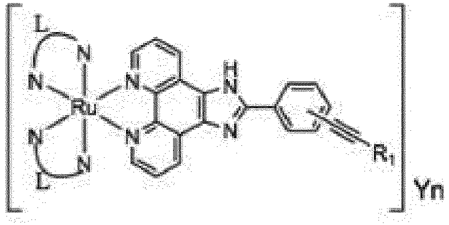 oder 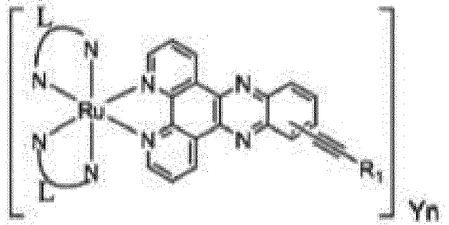 worin:
**L** ein Hilfsligand ist mit der Strukturformel: 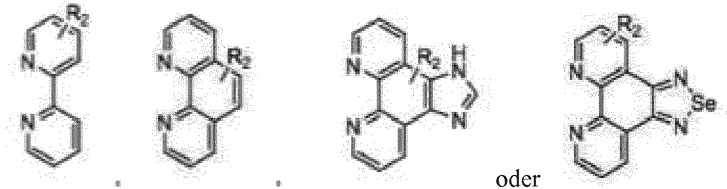
**R₁** unabhängig ausgewählt ist aus H, Hydroxyl, Trimethylsilyl, C₁-C₆ Alkyl oder substituiertem C₁-C₆ Alkyl, Phenyl oder substituiertem Phenyl, Pyridyl oder substituiertem Pyridyl, Furyl oder substituiertem Furyl, Pyrryl oder substituiertem Pyrryl, Thiazolyl oder substituiertem Thiazolyl; wobei die Position von R₁-substituiertem Ethinyl die ortho-Position, meta-Position oder para-Position am Benzolring sein kann; die Anzahl der substituierten Ethinylgruppen 1,2 oder mehr beträgt;
**R₂** unabhängig ausgewählt ist aus H, Methyl, Methoxyl, Nitro und Halogen;
**Y** ein Ion oder saures Radikalion ist, das den gesamten Ruthenium(II)-Komplex elektrisch neutral macht,
**n** die Anzahl der Ionen oder sauren Radikalionen ist, die den gesamten Ruthenium(II)-Komplex elektrisch neutral macht; und die Länge der Nukleinsäuresequenz mindestens 4 bp beträgt,
und wobei die Nukleinsäure ausgewählt ist aus DNA der c-myc Promotorregion, DNA der C-kit Promotorregion, DNA der bcl-2 Promotorregion, miR-21, DNA von AS1411, siRNA, microRNA, Aptamer und mRNA;
wobei die Nukleinsäure eine Fluorophor-markierte Nukleinsäure ist;
wobei die Ruthenium-Komplexe als Nukleinsäurevektoren eingesetzt werden, und
wobei die Nukleinsäuresequenzen zur Fluoreszenzverfolgung in Zellen abgegeben werden.

2. Nichttherapeutische Anwendung von Ruthenium-Nukleinsäure-Komplexen nach Anspruch 1, wobei der Ruthenium-Komplex ausgewählt ist aus:

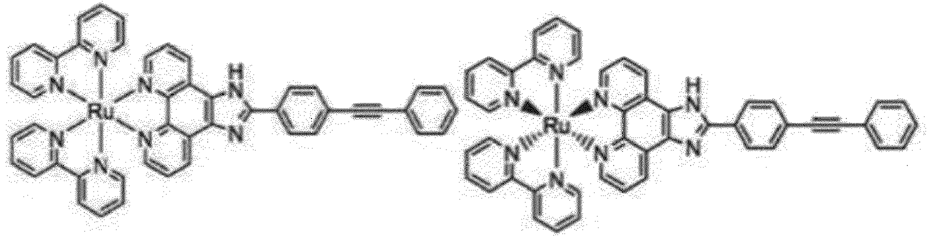

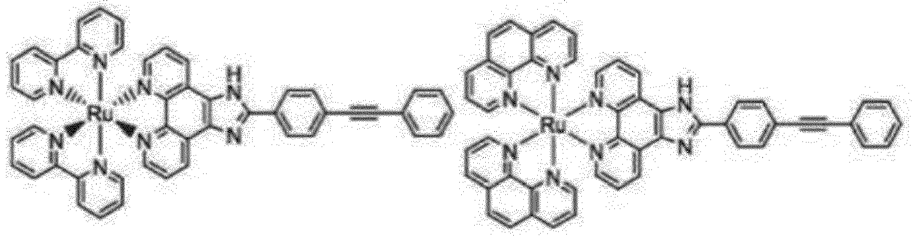

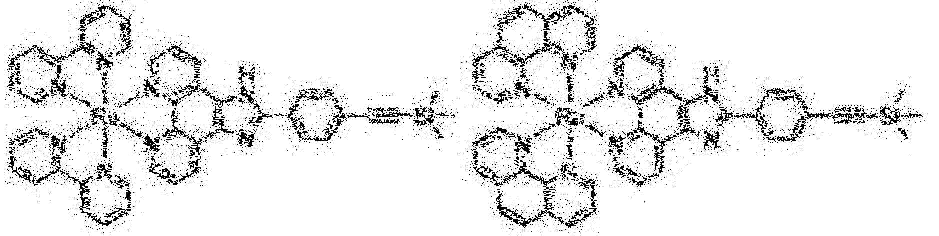

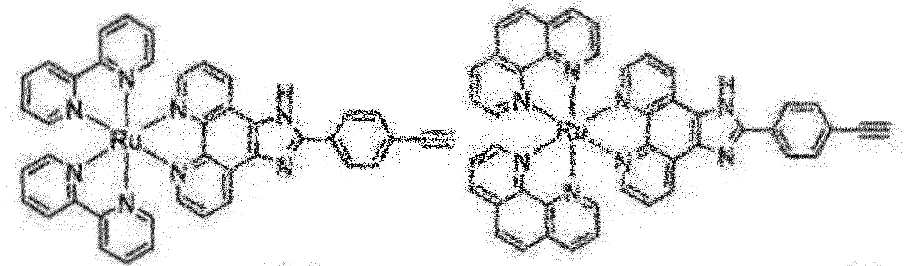

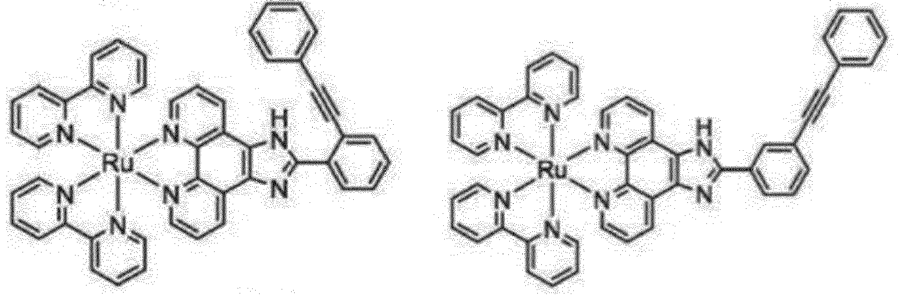

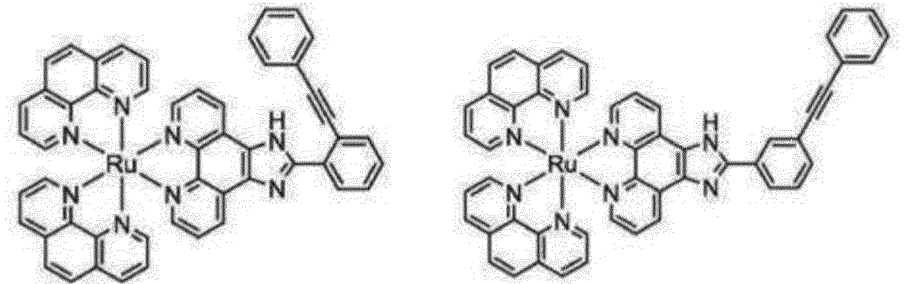

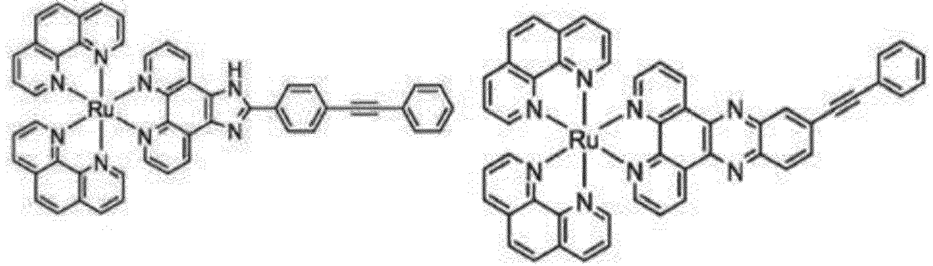

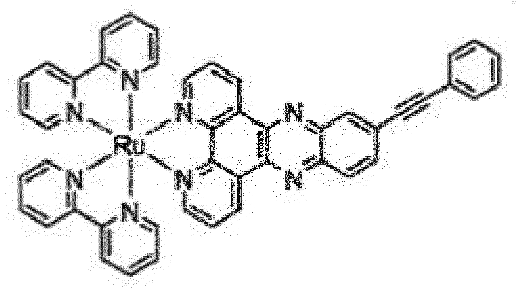

3. Nichttherapeutische Anwendung von Ruthenium-Nukleinsäure-Komplexen nach Anspruch 1 oder 2, **dadurch gekennzeichnet, dass** der Ruthenium-Komplex ein einzelnes chirales Isomer davon ist.

4. Nichttherapeutische Anwendung von Ruthenium-Nukleinsäure-Komplexen nach Anspruch 1, **dadurch gekennzeichnet, dass** die Länge der Nukleinsäure nicht länger als 3.000 bp ist.

5. Verfahren zur Herstellung eines Ruthenium-Koordinationskomplex-Nukleinsäure-Komplexes, das die folgenden Schritte umfasst:
der Ruthenium-Komplex wird gleichmäßig mit der Nukleinsäurelösung vermischt, dann wird das erhaltene Gemisch auf eine Temperatur im Bereich von 70 bis 100 °C erhitzt und 30 s bis 30 min lang bei dieser Temperatur gehalten;
freie Ruthenium-Komplexe werden nach dem Abkühlen entfernt, um einen Ruthenium-Koordinationskomplex-Nukleinsäure-Komplex zu bilden;
wobei der Rutheniumkomplex eine Strukturformel nach einem der Ansprüche 1-3 aufweist und die Länge der Nukleinsäure nicht kürzer als 4 bp ist, und wobei die Nukleinsäure ausgewählt ist aus DNA der c-myc Promotorregion, DNA der C-kit Promotorregion, DNA der bcl-2-Promotorregion, miR-21, DNA von AS1411, siRNA, microRNA, Aptamer und mRNA;
wobei die Nukleinsäure eine Fluorophor-markierte Nukleinsäure ist.

6. Verfahren nach Anspruch 5, **dadurch gekennzeichnet, dass** das Erhitzen durch eine Mikrowelle erfolgt.

## Revendications

1. Application non thérapeutique de complexes d'acides nucléiques à base de ruthénium, dans laquelle le complexe de ruthénium répond à l'une des formules générales suivantes : 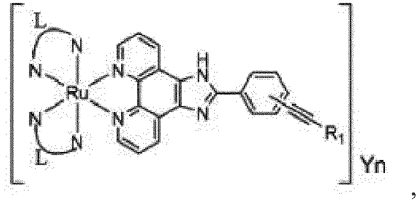 ou 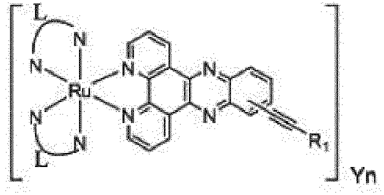 dans laquelle :
L représente un ligand auxiliaire répondant à une formule structurelle de : 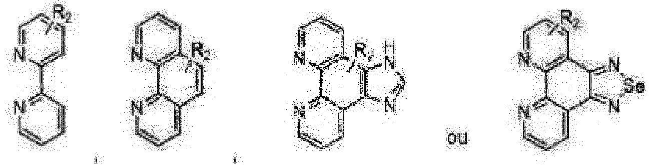
**R₁** est indépendamment choisi parmi H, hydroxyle, triméthylsilyle, alkyle en C1-C5 ou alkyle en C1-C5 substitué, phenyle ou phenyle substitué, pyridyle ou pyridyle substitué, furyle ou furyle substitué, pyrryle ou pyrryle substitué, thiazolyle ou thiazolyle substitué ; dans laquelle la position du groupe éthynyle substitué par R1 peut être une position ortho, une position méta ou une position para sur le cycle benzénique ; le nombre d'éthynyle substitué est de 1, 2 ou plus ;
**R²** est indépendamment choisi parmi H, méthyle, méthoxyle, nitro et halogène ;
**Y** est un ion ou un ion radicalaire acide qui rend la totalité du complexe de ruthénium (II) électriquement neutre,
**n** est le nombre d'ions ou d'ions radicalaires acides qui rend la totalité du complexe de ruthénium (II) électriquement neutre ; et la longueur de la séquence d'acides nucléiques est d'au moins 4 bp,
et dans laquelle l'acide nucléique est choisi parmi un ADN de la région de promoteur c-myc, un ADN de la région de promoteur C-kit, un ADN de la région de promoteur bcl-2, le miR-21, un ADN de AS1411, un ARNsi, un microARN, un aptamère et un ARNm ; dans laquelle l'acide nucléique est un acide nucléique marqué au fluorophore ;
dans laquelle les complexes de ruthénium sont employés comme des vecteurs d'acide nucléique, et
dans laquelle les séquences d'acides nucléiques sont délivrées dans des cellules pour un suivi fluorescent.

2. Application non thérapeutique de complexes d'acides nucléiques à base de ruthénium selon la revendication 1, dans laquelle le complexe de ruthénium est choisi parmi :

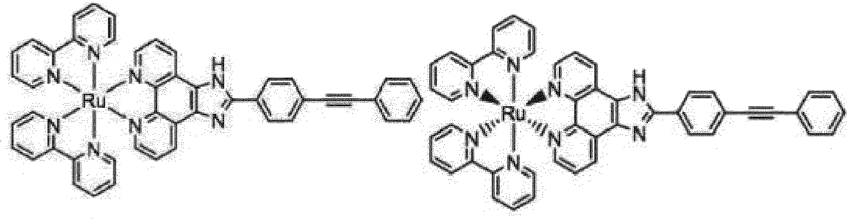

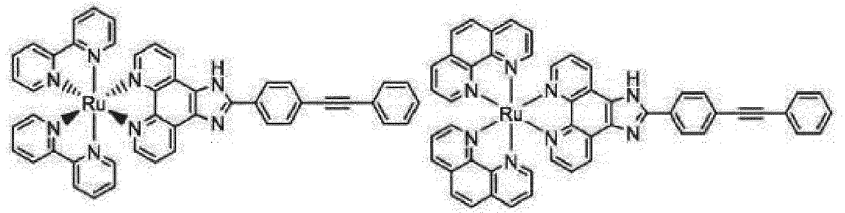

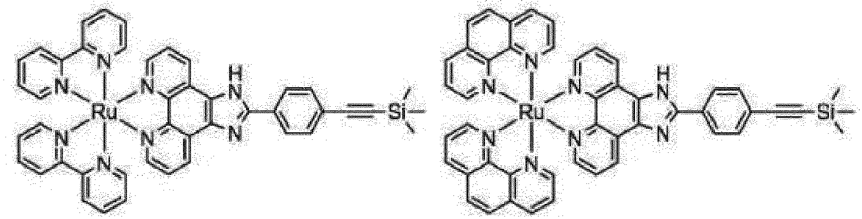

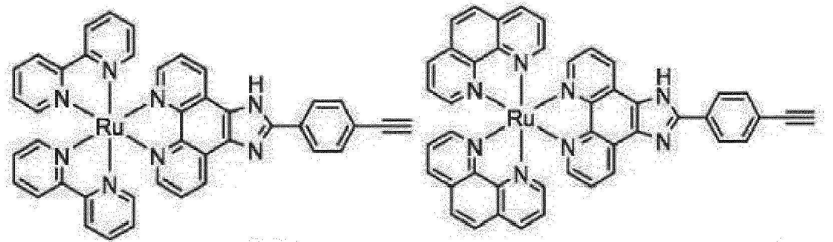

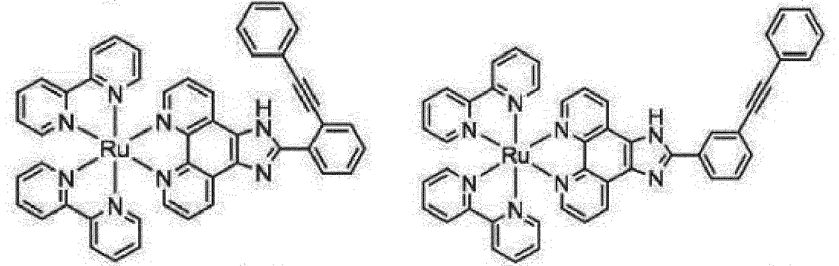

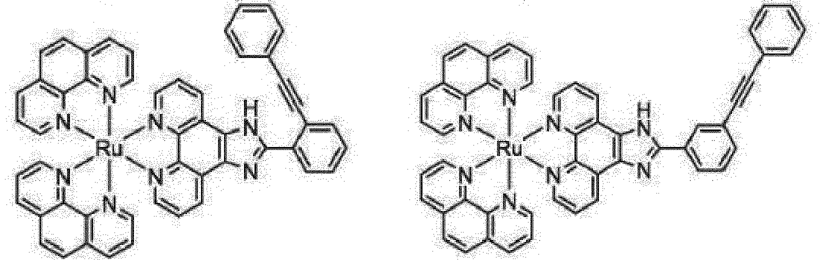

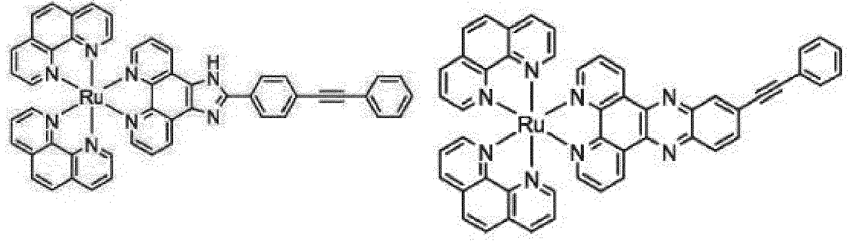

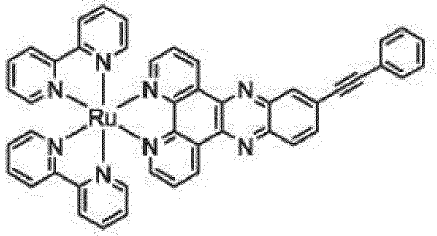

3. Application non thérapeutique de complexes d'acides nucléiques à base de ruthénium selon la revendication 1 ou la revendication 2, qui est **caractérisée en ce que** le complexe de ruthénium est un isomère chiral unique de celui-ci.

4. Application non thérapeutique de complexes d'acides nucléiques à base de ruthénium selon la revendication 1, qui est **caractérisée en ce que** la longueur de l'acide nucléique n'est pas supérieure à 3000 bp.

5. Procédé de préparation d'un complexe de coordination au ruthénium - d'un complexe d'acides nucléiques, comprenant les étapes suivantes :
le complexe de ruthénium est mélangé de manière uniforme avec la solution d'acides nucléiques, ensuite le mélange obtenu est chauffé à une température qui va de 70 à 100 °C et est maintenu à la température pendant 30 secondes à 30 minutes ;
les complexes exempts de ruthénium sont éliminés après refroidissement afin de former un complexe de coordination au ruthénium - un complexe d'acides nucléiques ;
dans lequel le complexe de ruthénium comporte une formule structurelle selon l'une quelconque des revendications 1 à 3 et la longueurs de l'acide nucléique n'est pas inférieure à 4 bp, et dans lequel l'acide nucléique est choisi parmi un ADN de la région de promoteur c-myc, un ADN de la région de promoteur C-kit, un ADN de la région de promoteur bcl-2, le miR-21, un ADN de AS1411, un ARNsi, un microARN, un aptamère et un ARNm ;
dans lequel l'acide nucléique est un acide nucléique marqué au fluorophore.

6. Procédé selon la revendication 5, qui est **caractérisé en ce que** le chauffage est effectué par un micro-ondes.
